(19) Europäisches Patentamt
European Patent Office
Office européen des brevets

(11) EP 2 712 605 A1

(12) EUROPEAN PATENT APPLICATION

(43) Date of publication:
**02.04.2014 Bulletin 2014/14**

(21) Application number: **13176937.4**

(22) Date of filing: **22.03.2011**

(51) Int Cl.:
***A61K 8/31*** *(2006.01)* ***A61K 8/73*** *(2006.01)*
***A61K 8/81*** *(2006.01)* ***A61K 8/85*** *(2006.01)*
***A61K 8/891*** *(2006.01)* ***A61K 8/893*** *(2006.01)*
***A61K 8/90*** *(2006.01)* ***A61Q 1/10*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**29.03.2010 FR 1052264**
**29.03.2010 FR 1052263**
**29.03.2010 FR 1052266**
**14.04.2010 US 323898 P**
**14.04.2010 US 323896 P**
**14.04.2010 US 323899 P**

(62) Document number(s) of the earlier application(s) in accordance with Art. 76 EPC:
**11712499.0 / 2 552 386**

(71) Applicant: **L'Oréal**
**75008 Paris (FR)**

(72) Inventors:
- **Pays, Karl**
  **94410 Saint Maurice (FR)**
- **Barba, Claudia**
  **75010 Paris (FR)**
- **Le Merrer, Carole**
  **92120 Montrouge (FR)**

(74) Representative: **Goudet, Sylvain**
**L'Oréal**
**D.I.P.I.**
**25-29 Quai Aulagnier**
**92665 Asnières-sur-Seine Cedex (FR)**

Remarks:
This application was filed on 17-07-2013 as a divisional application to the application mentioned under INID code 62.

# (54) Composition for making up the eyelashes or eyebrows, combination and methods

(57) A subject-matter of the present invention is a composition for making up keratinous fibres, comprising:
- at least one compound or a mixture of compounds capable of conferring, on the said composition, a dmax threading nature of greater than or equal to 5 mm,
- at least one silicone polymer, in particular glycerolated silicone polymer.

Another subject-matter of the present invention is a method for making up keratinous fibres and also a packaging and applicational combination for a composition for making up keratinous fibres.

EP 2 712 605 A1

## Description

The present invention relates to a composition for making up keratinous substances which is capable of forming an elongating effect under hot conditions on keratinous fibres, such as the eyelashes or eyebrows, and which can be easily removed. It also relates to a combination or kit and methods of a specific nature.

The make-up composition can be provided in the form of a mascara or of a product for the eyebrows. More preferably, the invention relates to the removal and/or the cleansing of a mascara. The term "mascara" is understood to mean a composition intended to be applied to the eyelashes: it can be a composition for making up the eyelashes or alternatively a composition for the cosmetic treatment of the eyelashes. The mascara is more particularly intended for human eyelashes but also for false eyelashes.

The compositions for making up the eyelashes or mascaras can be in anhydrous form or in the form of an emulsion, for example a water-in-oil or oil-in-water emulsion. They generally exhibit a pasty or solid texture and are packaged in a reservoir. An applicator is generally provided in order to apply, and optionally remove, the said make-up composition. This applicator can be provided in particular in the form of a brush or of a comb suitable for being brought into contact with the keratinous fibres to be made up, such as the eyelashes or eyebrows.

The prior art describes mascara compositions comprising fibres in order to obtain an elongating effect on the eyelashes. These fibres can add a small amount of physical length to the eyelashes when they are sufficiently rigid and visible and when they occur at the end of the eyelash. However, the gain in physical elongation obtained via such mascaras remains moderate as it is difficult to orient the fibres in order to stack them at the end of the eyelash. Furthermore, the presence of fibres can reduce the adhesion of mascara to the eyelashes, lengthening the time necessary for making up.

Another technical route described in the document EP 1 430 868 is the use of mascaras exhibiting a "threading" nature at ambient temperature and which are capable of forming, when applied to keratinous fibres and after drawing out using a brush, threads in the extension of the eyelashes, without use of a heat source.

However, the ability of these mascaras to form threads at ambient temperature does not simplify their use: in particular, when it is withdrawn in order to be applied, the mascara can form threads between the container in which it is present and the applicator or between the eyelashes and the applicator.

Moreover, control of the length of the threads formed on the eyelashes is problematic as the threads do not spontaneously break. In addition, the latter rarely exhibit a stiffness sufficient to remain aligned in the extension of the eyelash and to make possible a lasting elongating effect.

The aim of the present invention is to provide a method for coating keratinous fibres which makes possible a good elongating effect of the eyelashes, in particular under the action of heat, and good hold over time.

The properties described above can be obtained by using a composition exhibiting a specific threading nature under the action of a heat source, as described in application FR 2 905 068.

This type of composition generally has good properties of freedom from transfer and good properties of hold over time, in particular towards water, sebum and rubbing actions. Thus, this formulation route makes possible, in addition to a noteworthy elongating effect, an improvement in the performance of products for making up, caring for or treating keratinous substances.

On the other hand, these compositions are more difficult to remove from the made-up keratinous fibres than conventional products.

One aim of the present invention is thus the development of a make-up composition which, while retaining an elongating effect and good hold on the eyelashes or eyebrows, furthermore exhibits a good ability to be removed.

To do this, a subject-matter of the present invention is, according to a first aspect, a composition for making up keratinous fibres, such as the eyelashes or eyebrows, with improved make-up-removing properties, comprising:

- at least one compound or one mixture of compounds capable of conferring, on the said composition, a dmax threading nature of greater than or equal to 5 mm,
- at least one noncyclic silicone oil of following general formula (I):

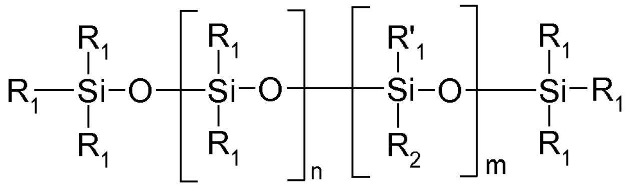

(I)

with:

○ **$R_1$**, which are identical or different, representing:

- i) a linear or branched ($C_1$-$C_{20}$)alkyl group, particularly a linear or branched $C_1$-$C_6$ alkyl group, such as a methyl, ethyl, propyl or butyl group; or
- ii) a hydroxyl group;

○ **$R_2$** representing:

- i) a linear or branched ($C_1$-$C_{20}$)alkyl group which is optionally interrupted and/or terminated by a heteroatom, such as O, S or N; in particular, i) is a linear or branched $C_1$-$C_6$ alkyl group, such as a methyl, ethyl, propyl or butyl group;
- ii) a (poly)halo($C_1$-$C_9$)alkyl group, in particular a perfluoroalkyl group, comprising from 1 to 9 halogen atoms, particularly fluorine, such as trifluoromethyl; and
- iii) the polysiloxane group $-O-[Si(R_1)_2-O]_{n'}-Si(R_1)_3$, with $R_1$ as defined above;

○ **$R'_1$** representing an $R_1$ or $R_2$ radical as defined above;
○ **m** being an integer inclusively between 0 and 150, preferably 20 and 100;
○ **n** and **n'**, which are identical or different, being an integer inclusively between 1 and 300, preferably 1 and 100;

- the said oil exhibiting a viscosity at 25°C of less than or equal to 300 cSt.

The compound or a mixture of compounds capable of conferring, on the said composition, a dmax threading nature of greater than or equal to 5 mm and the noncyclic silicone oil are formulated together. In other words, the said compound and the said oil are provided in the same composition. They are thus packaged together.
Another subject-matter of the present invention is a packaging and applicational combination for a composition for making up and/or caring for keratinous fibres, in particular the eyelashes or eyebrows, comprising:

- a reservoir,
- a composition for making up and/or caring for keratinous fibres as defined above, positioned inside the reservoir, and
- a device for the application of the make-up and/or care composition.

Such a combination can additionally comprise a heating device in order to bring, prior to, simultaneously with or subsequent to the application thereof, the said composition to a temperature of greater than or equal to 40°C. This heating device can optionally be carried, at least in part, better still completely, by the applicational device.
A further subject-matter of the invention is a cosmetic method for making up and/or for the nontherapeutic care of keratinous fibres, comprising:

- a first stage of application, to the keratinous fibres, of a cosmetic composition comprising at least one compound or one mixture of compounds which, when the composition is brought to a temperature of greater than or equal to 40°C, confèrs, on the said composition, a dmax threading nature of greater than or equal to 5 mm, the said composition being brought, prior to, simultaneously with or subsequent to the application thereof, to a temperature of greater than or equal to 40°C, and
- a second stage of removing the said composition by means of a make-up-removing and/or cleansing composition or a make-up-removing substrate, optionally (pre)impregnated with the said make-up-removing and/or cleansing composition.

Another subject-matter of the present invention is a method for removing make-up from and/or cleansing keratinous fibres, such as the eyelashes or eyebrows, comprising:

- a make-up composition as defined above,
- the said method comprising a stage which consists in applying, to the make-up film, a make-up-removing and/or cleansing composition or a make-up-removing substrate, optionally (pre)impregnated with the said make-up-removing and/or cleansing composition.

Another subject-matter of the present invention is a combination or kit for removing make-up from keratinous fibres, such as the eyelashes or eyebrows, comprising:

- at least one make-up composition described above, and
- at least one make-up-removing and/or cleansing substrate or composition.

The make-up-removing stage can optionally consist in pulling on the make-up film from the base of the keratinous fibre to the free end of the said fibre for the purpose of at least partially removing the said film. This stage can be carried out manually and in particular using a woven or nonwoven and optionally laminated substrate, such as a Demak'up® disc. This substrate can be anhydrous. In an alternative form, this substrate can be preimpregnated with the make-up-removing and/or cleansing composition.

This make-up-removing and/or cleansing composition can be water or a water-soluble solvent, such as, for example, the Effacil® product from Lancôme. The make-up-removing and/or cleansing composition can comprise essentially water and/or one or more water-soluble solvent(s), for example present in an amount of greater than or equal to 95% by weight, with respect to the total weight of the composition, and in particular ranging from 80 to 100% by weight, with respect to the total weight of the composition.

In an alternative form, and preferably, this composition can comprise an oily phase, such as the Bifacil® product from Lancôme. The make-up-removing and/or cleansing composition can comprise an oily phase present in an amount of greater than or equal to 95% by weight, with respect to the total weight of the composition, and in particular ranging from 80 to 100% by weight, with respect to the total weight of the composition. Such a composition can optionally consist of a mixture of an oily phase with an aqueous phase comprising water and/or one or more water-soluble solvent(s).

The inventors have discovered that these specific make-up films can be easily removed by a conventional pulling movement. This make-up removing can be carried out sheathwise, without substantial disintegration of the make-up films. Such a make-up removing can then prevent the make-up composition from being dispersed during the removal thereof from the keratinous fibres.

The term "sheaths" is understood to mean the formation of sleeves with a length of greater than or equal to 1 mm, better still of at least 2 mm.

The make-up composition according to the invention comprises a physiologically acceptable medium, that is to say a nontoxic medium capable of being applied to keratinous fibres, such as the eyelashes, eyebrows and hair of human beings, in particular compatible with the ocular region. This composition is preferably a mascara.

The composition can, for example, comprise at least one compound chosen from polymers and copolymers comprising at least one alkene monomer, in particular ethylene-based copolymers, and poly(vinyl acetate) homopolymers. Such polymers are particularly well suited to producing the desired threading. The content by weight of such compounds can be between 5 and 80%, preferably between 10 and 70%, with respect to the total weight of the product, before application.

The composition can be provided in the solid, semisolid or liquid form.

The composition can be provided in particular in the form of a suspension, dispersion, solution, gel, emulsion, in particular oil-in-water (O/W), wax-in-water, water-in-oil (W/O) or multiple (W/O/W or polyol/O/W or O/W/O) emulsion, cream, foam, dispersion of vesicles, in particular of ionic or nonionic lipids, two-phase or multiphase lotion, spray, powder, or paste, in particular soft paste. Each composition is preferbly a leave-in composition.

The composition according to the invention can be manufactured by known processes generally used in the cosmetics field.

According to one embodiment, the composition is in the solid form. According to a specific embodiment, the composition according to the invention is **characterized in that** the hardness in shearing is between 375 g/m and 15 000 g/m, in particular between 5000 g/m and 15 000 g/m.

According to a second aspect, considered independently of or in combination with the characteristics recited in connection with the first aspect, another subject-matter of the present invention is a composition for making up keratinous fibres, such as the eyelashes or eyebrows, with improved make-up-removing properties, comprising:

- at least one compound or one mixture of compounds capable of conferring, on the said composition, a dmax threading nature of greater than or equal to 5 mm of the same type as defined above, and
- at least one silicone polymer corresponding to the following general formula (I'):

$$R^1_a R^2_b R^3_c SiO_{(4-a-b-c)/2} \qquad (I')$$

in which formula (I'):

- a, b and c are such that a varies from 1 to 2.5, and b and c, independently of one another, vary from 0.001 to 1.5;
- $R^1$, identical or different, is chosen from:
- $C_1$ to $C_{30}$ alkyl radicals, if appropriate substituted by one or more fluorine atoms, amino groups and/or carboxyl groups,
- aryl or aralkyl radicals,
- radicals of general formula (II'):

$$-C_dH_{2d}-O-(C_2H_4O)_e(C_3H_6O)_fR^4 \qquad (II')$$

with:

- $R^4$ being a $C_1$ to $C_{30}$ hydrocarbon radical or an $R^5$-(CO)- radical with $R^5$ being a $C_1$ to $C_{30}$ hydrocarbon radical, and
- d, e and f being integers such that d varies from 0 to 15, and e and f, independently of one another, vary from 0 to 50, and
- their combinations,
- $R^2$ is a radical represented by the general formula (III'):

$$-Q-O-X \qquad (III')$$

with:

- Q being a divalent $C_2$ to $C_{20}$ hydrocarbon radical which can include at least one ether bond and/or at least one ester bond, and
- X being a polyhydroxylated hydrocarbon radical, in particular a polyhydroxy($C_1$-$C_6$)alkyl radical, such as $-[CH_2-CH(OH)-CH_2-O]_p-H$ with p between 1 and 10, preferably equal to 3,
- $R^3$ is an organosiloxane group of general formula (IV'):

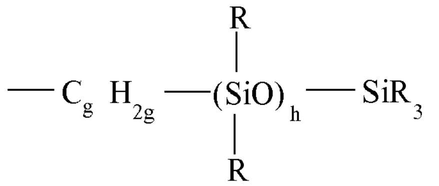

(IV')

with:

- each of the R radicals representing, independently of one another, a radical chosen from $C_1$ to $C_{30}$ alkyl radicals, if appropriate substituted by one or more fluorine atoms, and aryl and aralkyl radicals,
- g and h being integers such that g varies from 1 to 5 and h varies from 0 to 500.

According to a specific embodiment, the said at least one silicone polymer corresponds to the following formula (VIII'):

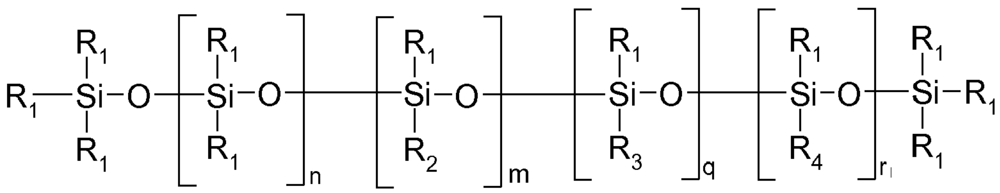

(VIII')

in which formula (VIII'):

○ **$R_1$**, which are identical or different, represent:

- i) a linear or branched ($C_1$-$C_{20}$)alkyl group, in particular a linear or branched $C_1$-$C_6$ alkyl group, such as methyl, ethyl, propyl or butyl; or
- ii) a hydroxyl group; preferably, **$R_1$** represents a methyl;

○ **$R_2$** represents the polysiloxane group $-ALK-[Si(R_1)_2-O]_{n'}-Si(R_1)_3$, with $R_1$ as defined above and ALK representing a divalent ($C_1$-$C_6$)alkylene group, such as methylene, ethylene or propylene, preferably ethylene;

○ **$R_3$** represents the $-ALK'-O-[CH_2-CH(OH)-CH_2-O]_p-H$ group, with p between 1 and 10 inclusive; preferably, p is equal to 3; and ALK' representing a divalent ($C_1$-$C_6$)alkylene group, such as methylene, ethylene or propylene, preferably propylene;

○ **$R_4$** represents a ($C_1$-$C_{20}$)alkyl group which is linear or branched, in particular one comprising from 8 to 16 carbon atoms, in particular one which is linear; preferably, one comprising 12 carbon atoms, such as lauryl;

○ **m** and **r**, which are identical or different, are an integer inclusively between 0 and 150, preferably 20 and 100;

○ **n, n'** and **q**, which are identical or different, are an integer inclusively between 1 and 300, preferably 1 and 100.

According to a specific embodiment, the said at least one silicone polymer corresponds to the following formula (IX') or (X'):

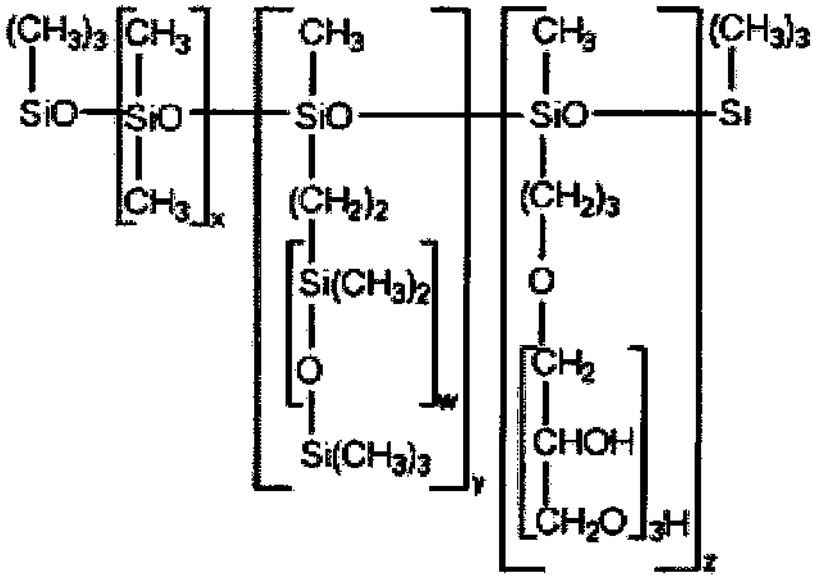

(IX')

in which formula (IX') x, y, w and z, which are identical or different, are an integer between 1 and 25 inclusive;

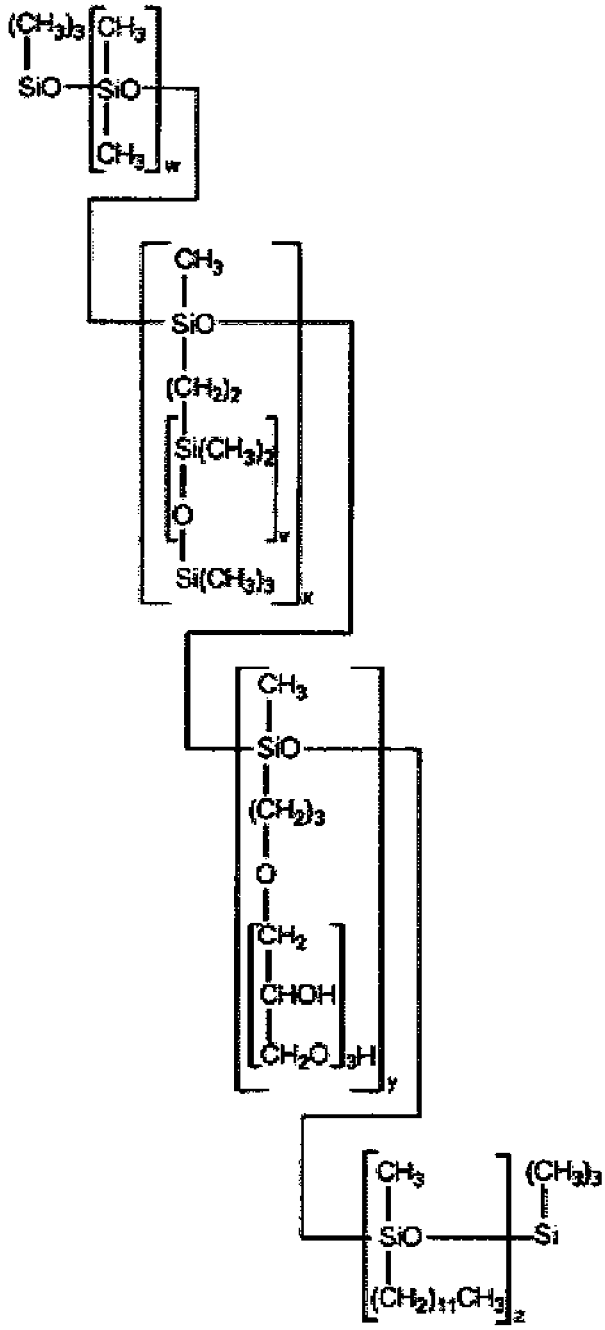

(X')

in which formula (X') x, y, w and z, which are identical or different, are an integer between 1 and 25 inclusive.

According to a third aspect, considered independently of or in combination with the characteristics recited in connection with the first aspect and/or the second aspect, another subject-matter of the present invention is a composition for making up keratinous fibres, such as the eyelashes or eyebrows, with improved make-up-removing properties, comprising:

- at least one compound or one mixture of compounds capable of conferring, on the said composition, a dmax threading nature of greater than or equal to 5 mm,
- at least one phenylated silicone oil exhibiting, at 25°C, a viscosity of greater than or equal to 400 cSt.

According to a specific embodiment, the said phenylated silicone oil is chosen from:

i) compounds of formula (I"):

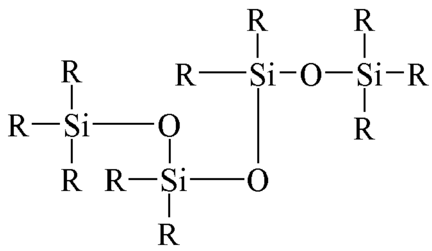

(I'')

in which the R groups independently represent a methyl group or a phenyl group, at least three of the R radicals being phenyl groups, indeed even at least four, in particular at least five;

ii) compounds of formula (II"):

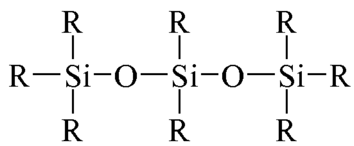

(II'')

in which the R groups independently represent a methyl group or a phenyl group, at least three of the R radicals being phenyl groups, indeed even at least four, in particular at least five;

iii) compounds of formula (III"):

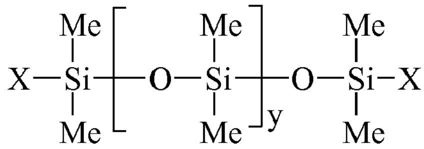

(III'')

in which X represents a $-CH_2-CH(CH_3)(Ph)$ group, Me represents a methyl group and Ph represents a phenyl group, and y varies between 1 and 10 000;

iv) compounds of formula (IV"):

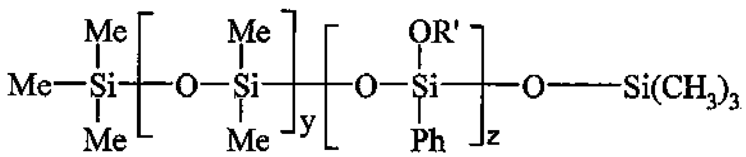

(IV'')

in which Me represents a methyl group and Ph represents a phenyl group, OR' represents an $-OSiMe_3$ group, y varies between 1 and 1000 and z varies between 1 and 1000;

v) compounds of formula (V"):

$$[R(CH_3)_2\text{-}SiO_{1/2}]_x[SiO_{4/2}]_y \qquad (V'')$$

in which R represents a phenylpropyl group and x and y vary, independently of one another, between 1 and 10 000;

vi) compounds of formula [VI"]:

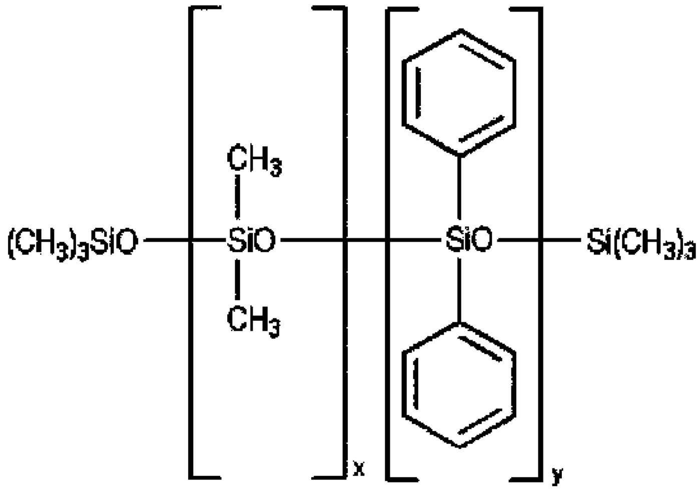

(VI")

in which x and y, which are identical or different, are both an integer varying, for each, from 1 to 50;
vii) compounds of formula (VII"):

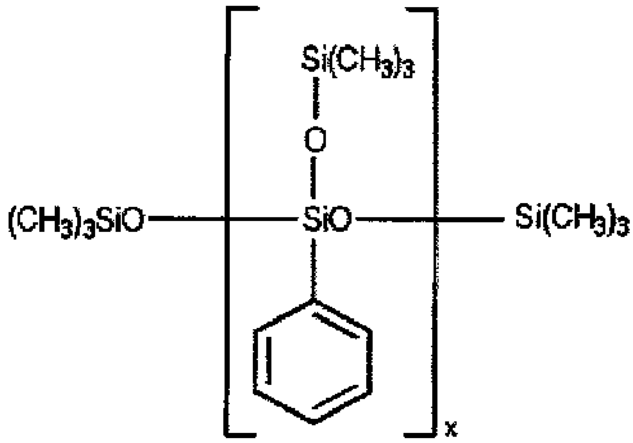

(VII")

in which x is an integer varying from 1 to 50.

Preferably, this phenylated silicone oil is non-volatile.

**Measurement of the hardness in shearing**

Use may be made, in order to determine the "hardness in shearing" of a piece of product in accordance with the invention, of the "cheesewire" method, which consists in transversely cutting a piece of product, in particular with a cylindrical surface or even a cylinder, for example with a diameter of 8 mm, using a stiff tungsten wire with a diameter of 250 μm, the wire being advanced at a rate of 100 mm/min with respect to the stick. The hardness corresponds to the maximum shear force exerted by the wire on the piece of product at 20°C, this force being measured using a DFGS2 tensile testing device sold by Indelco-Chatillom The measurement is repeated 6 times. The mean of the 6 values read using the tensile testing device mentioned above is expressed in grams. These values are thus between 3 g and 40 g, advantageously between 4 g and 20 g and preferably between 6 g and 12 g. This operation is carried out over the greatest length of the cross section of the piece of product. This value is then standardized in grams by this greatest length, for example by the diameter of the piece of product when it is cylindrical, i.e. 8 mm, for example, in order to obtain a value in grams/metre.

In other words, the value of the hardness corresponds to the maximum shear force exerted by the wire, measured on the stick at 20°C, divided by the greatest length of the cross section of the said piece of product brought into contact with the wire.

**Threading nature**

The threading nature represents the ability of the composition, once subjected to a heat source, to form, on the keratinous fibres, threads which, after drawing out using an applicator, are sufficiently consistent and retain their shape. The use of heat so as to bring the composition to a temperature greater than or equal to 40°C makes it possible to control the length of the threads formed in the extension of the eyelashes. In particular, after application of the softened composition and drawing out the threads, the latter solidify at ambient temperature in the extension of each eyelash and make it possible to obtain a noteworthy elongating effect.

The composition can in particular be used in combination with a heating instrument, such as a heating brush or comb, which can be applied to the eyelashes before, during or after the coating of the latter with the composition, or can be packaged in a device which makes it possible to apply the composition under warm conditions.

The composition can be brought to a temperature of greater than or equal to 40°C prior to, simultaneously with or subsequent to the application thereof, in particular using an applicational device comprising heating means, such as a heating brush or comb.

The composition employed according to the invention can be heated to a temperature of greater than or equal to 45°C, preferably of greater than or equal to 50°C, better still of greater than or equal to 55°C and even better still of greater than or equal to 65°C. The temperature can range up to 150°C, preferably up to 120°C, better still up to 100°C and even better still up to 95°C.

The compound or the mixture of compounds conferring, on the said composition, a dmax threading nature of greater than or equal to 5 mm can exhibit a thermoplastic behaviour.

**Measurement of the threading nature**

The threading nature of the composition is determined using the texture analyser sold under the name TA-XT2i by Rheo, equipped with a temperature-controlled spindle, this spindle being a heating cartridge made of stainless steel with the reference Firerod DIV-STL (Watlow, France), with a diameter of 3.17 mm and with a length of 60 mm, with a maximum power of 40W under a voltage of 24V, with a thermocouple of K loc C type.

The heating cartridge is supplied by a 5V/0.5A LKS 005-5V direct current source from Elka-Electronique. Its temperature is regulated by a PID TC48 controller from Faucigny Instrument (France). An attachment lengthening piece was created in order to attach the temperature-controlled spindle to the measuring arm of the texture analyser.

The measurement is performed on threads of composition obtained by applying a vertical displacement on the spindle until contact with a sample of the composition and then, after a waiting period in contact, by applying a vertical upwards displacement on the spindle. If the composition has a hot threading nature, a thread is formed between the spindle in the withdrawal phase and the sample of the composition, the said thread becoming firmer under the effect of the cooling in the surrounding air. The dmax measurement consists of a measurement of the length of the threads thus formed after detachment from the surface of the spindle.

The protocol is as follows:

a) a sample of the composition is prepared by filling, to its maximum, a pan made of stainless steel with a depth of 2 mm and a diameter of 20 mm, the excess composition being levelled at the surface,
b) the temperature of the spindle is controlled at 40°C,
c) the spindle descends at a rate of 10 mm/s until contact with the surface of the composition,
d) the spindle is held stationary for 10 s and is then raised again at a rate of 10 mm/s.

During the phase of withdrawal of the spindle, a thread is formed between the composition and the spindle. As the spindle is moved away from the surface of the composition, the thread formed cools and becomes firmer. From a certain elongation, the thread detaches from the spindle.

The threading nature or dmax (expressed in mm) corresponds to the length of the thread obtained after breaking, measured with a graduated ruler.

The measurement of the threading nature is repeated three times for the same composition, at different points in the pan, and a dmax "threading" mean is calculated for each composition.

Stages b) to d) are repeated for the same composition at a fixed spindle temperature in stage b) of 50°C, 60°C, 70°C, 80°C, 90°C, 100°C, 110°C, 120°C, 130°C and 140°C respectively.

Among the threading values which may be obtained at the various temperatures, the highest value is retained as dmax threading nature value.

Preferably, the composition is capable of forming a thread such that, if, after formation of the thread and measurement of the dmax according to the protocol indicated above, the pan comprising the composition is placed vertically (so that the thread is in a horizontal position, that is to say subjected to gravity) for at least 30 seconds, the

thread retains a minimum length of 5 mm (measurable manually with the graduated ruler).

The composition exhibiting such a threading nature according to the invention makes it possible to obtain, when applied to keratinous fibres, a thread of composition in the extension of the eyelash. This thread retains its shape, remains rigid and does not shrink, which makes it possible to obtain an elongating effect on the eyelash.

Preferably, the composition according to the invention is brought to the temperature at which it exhibits the dmax threading nature, measured as indicated above (that is to say, to the temperature at which the threading nature is highest).

The dmax can, for example, be greater than or equal to 7 mm, for example 10 mm, for example 15 mm. This dmax can be less than or equal to 200 mm, better still 150 mm, indeed even 100 mm.

**Compound capable of conferring, on the said composition, a dmax threading nature of greater than or equal to 5 mm**

The composition advantageously comprises at least one compound which confers, on the said composition, a dmax threading nature of greater than or equal to 5 mm or a mixture of compounds such that the said mixture confers, on the said composition, a dmax threading nature of greater than or equal to 5 mm, when the composition is heated to a temperature of greater than or equal to 40°C.

This compound can be a hydrocarbon or silicone compound and advantageously exhibits a thermoplastic behaviour.

This compound is preferably solid at ambient temperature. Advantageously, it itself exhibits a dmax threading nature of greater than or equal to 5 mm when it is brought to a temperature of greater than or equal to 40°C, that is to say that it is capable of producing threads as described above, at a temperature of greater than or equal to 40°C, for example ranging from 40 to 150°C, preferably of greater than or equal to 45°C, for example ranging from 45 to 120°C, better still of greater than or equal to 50°C, for example ranging from 50 to 100°C, and even better still of greater than or equal to 60°C.

This compound is preferably a polymer and can advantageously be chosen from:

A/ Polymers and copolymers comprising at least one alkene monomer, in particular ethylene-based copolymers.

Such compounds can be chosen from:

- copolymers of alkene and of vinyl acetate, in particular copolymers of ethylene and of vinyl acetate.
  Use is made in particular of the copolymers of ethylene and of vinyl acetate preferably comprising more than 25% by weight of vinyl acetate, with respect to the total weight of the polymer.
  Mention may be made, as example of ethylene/vinyl acetate copolymers, of those which are sold under the Elvax name by Du Pont de Nemours and in particular the compounds Elvax 40W, Elvax 140W, Elvax 200W, Elvax 205W, Elvax 210W and Elvax 310.
  Mention may also be made of the products sold under the Evatane name by Arkema, such as Evatane 28-800.
- copolymers of ethylene and of octene, such as, for example, the products sold under the "Affinity" reference by Dow Plastics, such as, for example, Affinity GA 1900 and GA 1950.

These polymers and copolymers can be used alone or as a mixture with at least one compound chosen from "tackifying" resins, such as described in the Handbook of Pressure Sensitive Adhesives, edited by Donatas Satas, 3rd ed., 1989, pp. 609-619, waxes, such as described later, and their combinations. The tackifying resins can in particular be chosen from rosin, rosin derivatives, hydrocarbon resins and their mixtures. Mention may in particular be made of indene hydrocarbon resins, such as the resins resulting from the polymerization predominantly of indene monomer with a minor proportion of monomers chosen from styrene, methylindene, methylstyrene and their mixtures. These resins can optionally be hydrogenated. They can exhibit a molecular weight ranging from 290 to 1150. Mention may in particular be made, as examples of indene resins, of the hydrogenated indene/methylstyrene/styrene copolymers sold under the "Regalite" name by Eastman Chemical, in particular Regalite R 1100, Regalite R 1090, Regalite R-7100, Regalite R1010 Hydrocarbon Resin and Regalite R1125 Hydrocarbon Resin.

Mention may be made, as mixture based on ethylene/vinyl acetate copolymer, for example of the products sold under the Coolbind name by National Starch.

These polymers can be provided in their pure form or can be conveyed in an aqueous phase or an organic solvent phase.

B/ Poly(vinyl acetate) homopolymers, preferably exhibiting a molecular weight of less than 20 000, such as, for example, Raviflex BL1S from Vinavil.

C/ Silicone resins

These resins are crosslinked organosiloxane polymers.
The nomenclature of silicone resins is known under the name of "MDTQ", the resin being described according to the various monomeric siloxane units which it comprises, each of the letters "MDTQ" characterizing one type of unit. The letter M represents the monofunctional unit of formula $(CH_3)_3SiO_{1/2}$, the silicon atom being connected to a single oxygen atom in the polymer comprising this unit.
The letter D means a difunctional unit $(CH_3)_2SiO_{2/2}$ in which the silicon atom is connected to two oxygen atoms.
The letter T represents a trifunctional unit of formula $(CH_3)SiO_{3/2}$.
In the M, D and T units defined above, at least one of the methyl groups can be replaced by a group R which is different from the methyl group, such as a hydrocarbon (in particular alkyl) radical having from 2 to 10 carbon atoms or a phenyl group or alternatively a hydroxyl group.
Finally, the letter Q means a tetrafunctional unit $SiO_{4/2}$ in which the silicon atom is bonded to four hydrogen atoms, themselves bonded to the remainder of the polymer.
Mention may in particular be made ofT resins, especially functionalized T silicone resins, such as polyphenylsiloxanes, especially functionalized by silanol (Si-OH) groups, such as that sold under the reference Dow Coming (R) Z-1806.

D/ Film-forming block ethylenic polymers

These polymers preferably comprise at least one first block and at least one second block having different glass transition temperatures (Tg), the said first and second blocks being connected to one another via an intermediate block comprising at least one constituent monomer of the first block and at least one constituent monomer of the second block.
Advantageously, the first and second blocks of the block polymer are incompatible with one another.
Such polymers are described, for example, in the documents EP 1 411 069, WO 04/028488 and WO 04/028493.
The term "block" polymer is understood to mean a polymer comprising at least 2 distinct blocks, for example at least 3 distinct blocks.
The first and second blocks of the polymer differ from one another in their degree of deformability. Thus, the first block can be rigid and the second block can be flexible.
The glass transition temperatures of the flexible and rigid blocks can be theoretical Tg values determined from the theoretical Tg values of the constituent monomers of each of the blocks, which can be found in a reference handbook, such as the Polymer Handbook, 3rd ed., 1989, John Wiley, according to the following relationship, referred to as the Fox Law:

$$1/Tg = \sum_i (\omega_i/Tg_i),$$

$\omega_i$ being the mass fraction of the monomer i in the block under consideration and $Tg_i$ being the glass transition temperature of the homopolymer of the monomer i.
Unless otherwise indicated, the Tg values indicated for the first and second blocks in the present patent application are theoretical Tg values.
The rigid block can have a Tg of greater than 20°C.
The flexible block can have a Tg of less than or equal to 20°C.
According to one embodiment, the copolymer comprises a first rigid block and a second flexible block.
Preferably, the proportion of the rigid block ranges from 20 to 90% by weight of the copolymer, better still from 30 to 90% by weight and even better still from 50 to 90% by weight.
Preferably, the proportion of the flexible block ranges from 5 to 75% by weight of the copolymer, preferably from 10 to 50% by weight and better still from 15 to 45% by weight.

Rigid block

In the context of the present invention, the rigid block or blocks are more particularly formed from the following

monomers:

- methacrylates of formula $CH_2=C(CH_3)-COOR_1$ in which $R_1$ represents an unsubstituted linear or branched $C_1$ to $C_4$ alkyl group, such as a methyl, ethyl, propyl or isobutyl group, or $R_1$ represents a $C_4$ to $C_{12}$ cycloalkyl group, such as an isobornyl group,
- acrylates of formula $CH_2=CH-COOR_2$ in which $R_2$ represents a tert-butyl group or a $C_4$ to $C_{12}$ cycloalkyl group, such as an isobornyl group,
- (meth)acrylamides of formula:

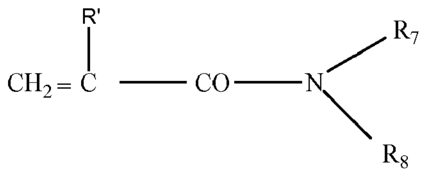

where $R_7$ and $R_8$, which are identical or different, each represent a hydrogen atom or a linear or branched $C_1$ to $C_{12}$ alkyl group, such as an n-butyl, t-butyl, isopropyl, isohexyl, isooctyl or isononyl group; or $R_7$ represents H and $R_8$ represents a 1,1-dimethyl-3-oxobutyl group,
and R' denotes H or methyl.
Mention may be made, as example of monomers of this type, ofN-butylacrylamide, N-(t-butyl)acrylamide, N-isopropylacrylamide, N,N-dimethylacrylamide and N,N-dibutylacrylamide,
- and their mixtures.

Particularly preferred monomers of the rigid block are isobornyl methacrylate, isobornyl acrylate and their mixtures.

Flexible block

In the context of the present invention, the flexible block or blocks are more particularly formed from the following monomers:

- acrylates of formula $CH_2=CHCOOR_3$, with $R_3$ representing an unsubstituted linear or branched $C_1$ to $C_{12}$ alkyl group, such as an isobutyl group (with the exception of a tert-butyl group), in which one or more heteroatoms chosen from O, N and S is/are optionally intercalated,
- methacrylates of formula $CH_2=C(CH_3)-COOR_4$, with $R_4$ representing an unsubstituted linear or branched $C_6$ to $C_{12}$ alkyl group in which one or more heteroatoms chosen from O, N and S is/are optionally intercalated;
- vinyl esters of formula $R_5-CO-O-CH=CH_2$ where $R_5$ represents a linear or branched $C_4$ to $C_{12}$ alkyl group;
- $C_4$ to $C_{12}$ alkyl vinyl ethers;
- and their mixtures.

Particularly preferred monomers of the flexible block are isobutyl acrylate.
Each of the blocks can comprise a minor proportion of at least one constituent monomer of the other block.
Thus, the first block can comprise at least one constituent monomer of the second block, and vice versa.
Each of the first and/or second blocks can comprise, in addition to the monomers indicated above, one or more other monomers, known as additional monomers, which are different from the main monomers mentioned above.
This additional monomer is chosen, for example, from:

a) hydrophilic monomers, such as:

- monomers possessing ethylenic unsaturation(s), other than acrylic acid, comprising at least one carboxylic or sulphonic acid functional group, such as, for example, methacrylic acid, crotonic acid, maleic anhydride, itaconic acid, fumaric acid, maleic acid, acrylamido-propanesulphonic acid, vinylbenzoic acid or vinylphosphonic acid, and the salts of these,
- monomers possessing ethylenic unsaturation(s) comprising at least one tertiary amine functional group, such as 2-vinylpyridine, 4-vinylpyridine, dimethylaminoethyl methacrylate, diethylaminoethyl methacrylate, dimethylaminopropylmethacrylamide and the salts of these,
- methacrylates of formula $CH_2=C(CH_3)-COOR_6$

in which $R_6$ represents a linear or branched alkyl group comprising from 1 to 4 carbon atoms, such as a methyl, ethyl, propyl or isobutyl group, the said alkyl group being substituted by one or more substituents chosen from hydroxyl groups (such as 2-hydroxypropyl methacrylate or 2-hydroxyethyl methacrylate) and halogen atoms (Cl, Br, I, F), such as trifluoroethyl methacrylate,

- methacrylates of formula $CH_2=C(CH_3)-COOR_9$,
  $R_9$ representing a linear or branched $C_6$ to $C_{12}$ alkyl group in which one or more heteroatoms chosen from O, N and S is/are optionally intercalated, the said alkyl group being substituted by one or more substituents chosen from hydroxyl groups and halogen atoms (Cl, Br, I, F);
- acrylates of formula $CH_2=CHCOOR_{10}$,
  $R_{10}$ representing a linear or branched $C_1$ to $C_{12}$ alkyl group substituted by one or more substituents chosen from hydroxyl groups and halogen atoms (Cl, Br, I and F), such as 2-hydroxypropyl acrylate and 2-hydroxyethyl acrylate, or $R_{10}$ representing a ($C_1$-$C_{12}$)alkyl-O-POE (polyoxyethylene) with repetition of the oxyethylene unit from 5 to 30 times, for example methoxy-POE, or $R_{10}$ representing a polyoxyethylene group comprising from 5 to 30 ethylene oxide units,

b) monomers possessing ethylenic unsaturation(s) comprising one or more silicon atoms, such as methacryloyloxypropyltrimethoxysilane or methacryloyloxypropyltris(trimethylsiloxy)silane,

- and their mixtures.

This or these additional monomers generally represent(s) an amount of less than or equal to 30% by weight, for example from 1 to 30% by weight, preferably from 5 to 20% by weight and more preferably from 7 to 15% by weight, of the total weight of the first and/or second blocks.
According to one embodiment, the copolymer can comprise at least one first block and at least one second block connected to one another via an intermediate segment comprising at least one constituent monomer of the first block and at least one constituent monomer of the second block.
Preferably, the intermediate block results essentially from constituent monomers of the first block and of the second block.
Advantageously, the intermediate segment comprising at least one constituent monomer of the first block and at least one constituent monomer of the second block of the copolymer is a random polymer.
Advantageously, the copolymer results essentially from monomers chosen from alkyl methacrylates, alkyl acrylates and their mixtures.
The term "essentially" is understood to mean, in that which precedes and in that which follows, comprising at least 85%, preferably at least 90%, better still at least 95% and even better still 100%.
As regards the acrylate and methacrylate esters, they can derive from the esterification of linear or branched, cyclic or aromatic $C_1$ to $C_{12}$ alcohols, in particular $C_4$ to $C_{10}$ alcohols.
Mention may in particular be made, by way of illustration and without implied limitation of these alcohols, of isoborneol.
According to one embodiment, the said copolymer comprises at least acrylate and methacrylate monomers deriving from the esterification of one and the same alcohol and in particular isoborneol.
Preferably, the film-forming linear block polymer comprises at least isobornyl acrylate monomers, at least isobornyl methacrylate monomers and at least isobutyl acrylate monomers.
According to an alternative embodiment, the block polymer can comprise at least:

- a rigid block, which is an isobornyl methacrylate/isobornyl acrylate copolymer, and
- a flexible block, which is an isobutyl acrylate copolymer.

More specifically, the copolymer can comprise from 50 to 80% by weight of isobornyl methacrylate/acrylate and from 10 to 20% by weight of isobutyl acrylate.
The weight-average molecular weight (Mw) of the copolymer preferably ranges from 80 000 to 300 000, indeed even from 100 000 to 150 000. The number-average molecular weight (Mn) of the copolymer preferably ranges from 20 000 to 90 000; for example, it ranges from 25 000 to 45 000.

E/ Copolymers of dienes and of styrene, in particular copolymers of butadiene and of styrene.

Mention may in particular be made of the styrene/butadiene copolymers sold under the Pliolite S5E reference by Eliokem.

F/ Polyesters comprising at least one monomer carrying at least one $-SO_3M$ group (M representing a hydrogen atom, an ammonium ion $NH_4^+$ or a metal ion), also known as sulphopolyesters.

These polyesters advantageously have a glass transition temperature (Tg) of greater than 38°C.

They can exhibit a weight-average molecular weight advantageously of less than 200 000, for example ranging from 10 000 to 50 000.

These polyesters can be obtained in a known way by polycondensation of at least one dicarboxylic acid with at least one polyol, in particular diols. The dicarboxylic acid can be aliphatic, alicyclic or aromatic. Mention may be made, as example of such acids, of: oxalic acid, malonic acid, dimethylmalonic acid, succinic acid, glutaric acid, adipic acid, pimelic acid, 2,2-dimethylglutaric acid, azelaic acid, suberic acid, sebacic acid, fumaric acid, maleic acid, itaconic acid, phthalic acid, dodecanedioic acid, 1,3-cyclohexanedicarboxylic acid, 1,4-cyclohexanedicarboxylic acid, isophthalic acid, terephthalic acid, 2,5-norbomanedicarboxylic acid, diglycolic acid, thiodipropionic acid, 2,5-naphthalenedicarboxylic acid or 2,6-naphthalenedicarboxylic acid. These dicarboxylic acid monomers can be used alone or as a combination of at least two dicarboxylic acid monomers. The choice is preferably made, among these monomers, of phthalic acid, isophthalic acid or terephthalic acid.

The diol can be chosen from aliphatic, alicyclic or aromatic diols. Use is preferably made of a diol chosen from: ethylene glycol, diethylene glycol, triethylene glycol, 1,3-propanediol, cyclohexanedimethanol or 4-butanediol.

Use may be made, as other polyols, of glycerol, pentaerythritol, sorbitol or trimethylolpropane.

Polyesteramides can be obtained analogously to the polyesters by polycondensation of diacids with diamines or aminoalcohols. Use may be made, as diamine, of ethylenediamine, hexamethylenediamine, meta-phenylenediamine or para-phenylenediamine. Use may be made, as aminoalcohol, of monoethanolamine.

The polyester comprises at least one monomer carrying at least one $-SO_3M$ group, with M representing a hydrogen atom, an ammonium ion $NH_4^+$ or a metal ion, such as, for example, an $Na^+$, $Li^+$, $K^+$, $Mg^{2+}$, $Ca^{2+}$, $Cu^{2+}$, $Fe^{2+}$ or $Fe^{3+}$ ion. Use may in particular be made of a bifunctional aromatic monomer comprising such an $-SO_3M$ group.

The aromatic ring system of the bifunctional aromatic monomer additionally carrying an $-SO_3M$ group as described above can be chosen, for example, from the benzene, naphthalene, anthracene, biphenyl, oxydiphenyl, sulphonyldiphenyl or methylenediphenyl ring systems. Mention may be made, as example of bifunctional aromatic monomer additionally carrying an $-SO_3M$ group, of: sulphoisophthalic acid, sulphoterephthalic acid, sulphophthalic acid or 4-sulphonaphthalene-2,7-dicarboxylic acid.

It is preferable to use copolymers based on isophthalate/sulphoisophthalate and more particularly copolymers obtained by condensation of diethylene glycol, cyclohexanedimethanol, isophthalic acid and sulphoisophthalic acid.

Such polymers are sold, for example, under the Eastman AQ® trade name by Noveon, such as, for example, Eastman AQ 38S.

G/ Waxes

The wax under consideration in the context of the present invention is generally a lipophilic compound which is solid at ambient temperature (25°C), which is or is not deformable, which exhibits a reversible solid/liquid change in state and which has a melting point of greater than or equal to 30°C which can range up to 100°C and in particular up to 90°C.

On bringing the wax to the liquid state (melting), it is possible to render it miscible with oils and to form a microscopically homogeneous mixture but, on bringing the temperature of the mixture back to ambient temperature, recrystallization of the wax in the oils of the mixture is obtained.

In particular, the waxes suitable for the invention can exhibit a melting point of greater than or equal to 45°C and in particular of greater than or equal to 55°C. Within the meaning of the invention, the melting point corresponds to the temperature of the most endothermic peak observed by thermal analysis (DSC) as described in Standard ISO 11357-3; 1999. The melting point of the wax can be measured using a differential scanning calorimeter (DSC), for example the calorimeter sold under the name "MDSC 2920" by TA Instruments. The measurement protocol is as follows:

A 5 mg sample of wax placed in a crucible is subjected to a first rise in temperature ranging from -20°C to 100°C at a heating rate of 10°C/minute, is then cooled from 100°C to -20°C at a cooling rate of 10°C/minute and, finally, is subjected to a second rise in temperature ranging from -20°C to 100°C at a heating rate of 5°C/minute. During the second rise in temperature, the variation in the difference in power absorbed by the empty crucible and by the crucible containing the sample of wax is measured as a function of the temperature. The melting point of the compound is the value of the temperature corresponding to the tip of the peak of the curve representing the variation in the difference in power absorbed as a function of the temperature.

The waxes capable of being used in the compositions according to the invention are chosen from waxes of animal, vegetable, mineral or synthetic origin, and their mixtures, which are solid at ambient temperature.

The waxes which can be used in the compositions according to the invention generally exhibit a hardness

ranging from 0.01 MPa to 15 MPa, in particular of greater than 0.05 MPa and especially of greater than 0.1 MPa. The hardness is determined by the measurement of the compressive force measured at 20°C using a texture analyser sold under the name TA-XT2 by Rheo, equipped with a stainless steel cylinder with a diameter of 2 mm which is displaced at the measuring rate of 0.1 mm/s and which penetrates the wax to a penetration depth of 0.3 mm.

The measurement protocol is as follows:

The wax is melted at a temperature equal to the melting point of the wax + 10°C. The molten wax is cast in a receptacle with a diameter of 25 mm and a depth of 20 mm. The wax is recrystallized at ambient temperature (25°C) for 24 hours, so that the surface of the wax is flat and smooth, and then the wax is stored at 20°C for at least 1 hour before measuring the hardness or the tack.

The spindle of the texture analyser is displaced at a rate of 0.1 mm/s and then penetrates the wax to a penetration depth of 0.3 mm. When the spindle has penetrated the wax to the depth of 0.3 mm, the spindle is held stationary for 1 second (corresponding to the relaxation time) and is then withdrawn at the rate of 0.5 mm/s.

The value of the hardness is the maximum compressive force measured divided by the surface area of the cylinder of the texture analyser in contact with the wax.

Mention may in particular be made, by way of illustration of the waxes suitable for the invention, of hydrocarbon waxes, such as beeswax, lanolin wax and Chinese insect waxes; rice bran wax, carnauba wax, candelilla wax, ouricury wax, esparto wax, berry wax, shellac wax, Japan wax and sumac wax; montan wax, orange and lemon waxes, microcrystalline waxes, paraffin waxes and ozokerite; polyethylene waxes, the waxes obtained by the Fischer-Tropsch synthesis and waxy copolymers, and their esters, waxes obtained by catalytic hydrogenation of animal or vegetable oils having linear or branched $C_8$-$C_{32}$ fatty chains, such as isomerized jojoba oil, hydrogenated sunflower oil, hydrogenated castor oil, hydrogenated coconut oil, hydrogenated lanolin oil and di(1,1,1-trimethylolpropane) tetrastearate, sold under the name of Hest 2T-4S® by Heterene.

Mention may also be made of silicone waxes or fluorinated waxes.

Use may also be made of the waxes obtained by hydrogenation of castor oil esterified with cetyl alcohol which are sold under the names of Phytowax Castor 16L64® and 22L73® by Sophim. Such waxes are described in Application FR-A-2 792 190.

Use may be made of a wax referred to as a "tacky wax", that is to say having a tack of greater than or equal to 0.1 N.s and a hardness of less than or equal to 3.5 MPa. Use may be made, as tacky wax, of a $C_{20}$-$C_{40}$ alkyl (hydroxystearyloxy)stearate (the alkyl group comprising from 20 to 40 carbon atoms), alone or as a mixture. Such a wax is sold in particular under the names "Kester Wax K 82 P®", "Hydroxypolyester K 82 P®" and "Kester Wax K 80 P®" by Koster Keunen.

H/ Fibres

The term "fibre" should be understood as meaning an object with a length L and a diameter D such that L is much greater than D, D being the diameter of the circle in which the cross section of the fibre is framed. In particular, the L/D ratio (or aspect ratio) is chosen within the range from 3.5 to 2500, in particular from 5 to 500 and more particularly from 5 to 150.

The fibres which can be used in the composition of the invention can be fibres of synthetic or natural and inorganic or organic origin. They can be short or long, individual or organized, for example plaited, and hollow or solid. They can have any shape and can in particular be circular or polygonal (square, hexagonal or octagonal) in cross section, according to the specific application envisaged. In particular, their ends are blunted and/or polished to prevent injury. In particular, the fibres have a length ranging from 1 μm to 10 mm, in particular from 0.1 mm to 5 mm and more particularly from 0.3 mm to 3.5 mm. Their cross section can be included within a circle with a diameter ranging from 2 nm to 500 μm, in particular ranging from 100 nm to 100 μm and more particularly ranging from 1 μm to 50 μm. The weight or count of the fibres is often given in denier or decitex and represents the weight in grams per 9 km of yarn. The fibres according to the invention can in particular have a count chosen within the range from 0.15 to 30 denier and especially from 0.18 to 18 denier. The fibres which can be used in the composition of the invention can be chosen from rigid or nonrigid fibres. They can be of synthetic or natural and inorganic or organic origin.

Furthermore, the fibres may or may not be surface treated, may or may not be coated and may or may not be coloured.

Mention may be made, as fibres which can be used in the composition according to the invention, of fibres which are not rigid, such as polyamide (Nylon®) fibres, or fibres which are rigid, such as polyimideamide fibres, for example those sold under the Kermel® or Kermel Tech® names by Rhodia, or poly(p-phenylene terephthalamide) (or aramid) fibres, sold in particular under the Kevlar® name by DuPont de Nemours.

According to one embodiment, the composition comprises, as compounds which confer, on the composition, a dmax threading nature of greater than or equal to 5 mm, a mixture of wax, in particular paraffin wax, especially in a content of greater than or equal to 90% by weight, with respect to the total weight of the composition, and fibres, especially

cellulose fibres, for example in a content of 1 to 5% by weight, with respect to the total weight of the composition.

The compound or the mixture of compounds which confers, on the composition, a dmax threading nature of greater than or equal to 5 mm can be present in the composition in a content of dry matter of at least 5% by weight, with respect to the total weight of the composition, for example ranging from 5 to 100% by weight, with respect to the total weight of the composition, preferably ranging from 10 to 100% by weight and better still from 12 to 100% by weight.

According to a preferred embodiment, the compound or mixture of compounds conferring, on the composition, a dmax threading nature of greater than or equal to 5 mm preferably comprises at least one compound chosen from polymers and copolymers comprising at least one alkene monomer, in particular ethylene-based copolymers, and poly(vinyl acetate) homopolymers. Such polymers are particularly well suited to producing the desired threading.

According to specific embodiments, the content by weight of the compound or mixture of compounds can range from 5 to 100% by weight, with respect to the total weight of the composition, preferably from 10 to 100% by weight, better still from 25 to 100% by weight, indeed even from 50 to 100% by weight or also from 80 to 100% by weight, with respect to the total weight of the composition. The content by weight of such compounds can, for example, be between 60 and 90% by weight, with respect to the total weight of the composition, before application.

## NONCYCLIC SILICONE OILS OF GENERAL FORMULA (I)

The present invention comprises at least one noncyclic silicone oil or a mixture of silicone oils, at least one of which is noncyclic.

The term "silicone oil" is understood to mean an organopolysiloxane.

"Noncylic" oil denotes a linear or branched oil not comprising any ring. In other words, the oil in accordance with the invention is devoid of "(hetero)aryl radical" and/or of "(hetero)cycloalkyl radical".

The noncyclic silicone oil is preferably nonvolatile.

The term "nonvolatile oil" is understood to mean an oil which remains on the keratinous fibre, at ambient temperature and atmospheric pressure, for at least several hours and which has in particular a vapour pressure of less than $10^{-3}$ mmHg (0.13 Pa). A nonvolatile oil can also be defined as having a rate of evaporation such that, under the conditions defined above, the amount evaporated after 30 minutes is less than 0.07 $mg/cm^2$.

Preferably, the molecular weight of the noncyclic silicone oil is between 500 and 100 000 g/mol.

The noncyclic silicone oils have a viscosity advantageously chosen within the range extending from 4 to 10 000 $mm^2/s$ at 25°C, preferably from 4 to 5000 $mm^2/s$, and better still from 4 to 1000 $mm^2/s$ and even better still from 4 to 200 $mm^2/s$.

According to the invention, the noncyclic silicone oils exhibiting, at 25°C, a viscosity of less than or equal to 300 cSt are particularly suitable for the make-up removing of cosmetic compositions comprising a compound or a mixture of compounds conferring, on the said composition, a dmax threading nature of greater than or equal to 5 mm. This viscosity, at 25°C, can, for example, be between 1 and 250 cSt and in particular between 5 and 150 cSt.

The method for measuring the viscosity used in the invention in order to characterize the silicone oils according to the invention can be "kinematic viscosity at 25°C raw product CID-012-01" or also "Viscosity Ubbelohde DIN 51562-1 PV04001 25°C".

The noncyclic silicone oil can exhibit a refractive index of greater than 1.3, in particular of less than 1.6.

The noncyclic silicone oils which can be used in the make-up compositions according to the present invention are represented by the following general formula (I):

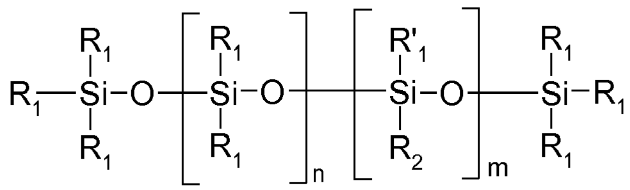

(I)

with:

○ $\mathbf{R_1}$, which are identical or different, representing:

- i) a linear or branched ($C_1$-$C_{20}$)alkyl group, particularly a linear or branched $C_1$-$C_6$ alkyl group, such as a methyl, ethyl, propyl or butyl group; or

- ii) a hydroxyl group;

○ $R_2$ representing:

- i) a linear or branched ($C_1$-$C_{20}$)alkyl group which is optionally interrupted and/or terminated by a heteroatom, such as O, S or N; in particular, i) is a linear or branched $C_1$-$C_6$ alkyl group, such as a methyl, ethyl, propyl or butyl group;
- ii) a (poly)halo($C_1$-$C_9$)alkyl group, in particular a perfluoroalkyl group, comprising from 1 to 9 halogen atoms, particularly fluorine, such as trifluoromethyl; and
- iii) the polysiloxane group $-O-[Si(R_1)_2-O]_{n'}-Si(R_1)_3$, with $R_1$ as defined above;

○ **R'$_1$** representing an $R_1$ or $R_2$ radical as defined above;
○ **m** being an integer inclusively between 0 and 150, preferably 20 and 100;
○ **n** and **n',** which are identical or different, being an integer inclusively between 1 and 300, preferably 1 and 100.

According to a preferred embodiment, $R'_1$ represents the $R_1$ radical and more particularly a ($C_1$-$C_6$)alkyl group, such as a methyl group.

According to a specific embodiment, m has the value 0.

According to another specific embodiment of the invention, $R_1$ is a methyl and more particularly m has a value 0 and $R_1$ is a methyl.

According to a specific example of the invention, the noncyclic silicone oils can be chosen from a fluorosilicone compound.

Mention may in particular be made, as fluorosilicone compounds, sold by Shin-Etsu under the names 'X22-819', 'X22-820', 'X22-821' and 'X22-822' or also 'FL-100'.

According to a preferred embodiment, the said noncyclic silicone oil is a dimethicone corresponding to the following formula (II):

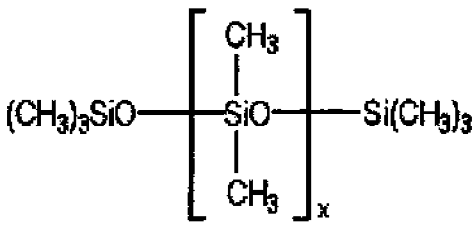

(II)

x being, in this formula (II), an integer varying from 1 to 50, better still from 1 to 20 and more specifically from 1 to 10. The molecular weight of such a compound can, for example, be approximately 770 g/mol. Preferably, x is equal to 8.

According to a specific embodiment, the noncyclic silicone oil of general formula (I) or (II) is advantageously chosen from the oils sold by Dow Corning under the reference 200R Fluid 5cSt® and under the reference 200R Fluid 100cSt®.

Such noncyclic silicone oils are particularly suitable for the make-up removal of cosmetic compositions comprising a compound or a mixture of compounds conferring, on the said composition, a dmax threading nature of greater than or equal to 5 mm.

Preferably, the noncyclic silicone oil in accordance with the invention is present in the composition according to the invention in a content ranging from 0.1 to 40% by weight, particularly from 0.5 to 35% by weight, for example from 2 to 25% by weight, better still from 5 to 20% by weight, indeed even from 8 to 16% by weight, with respect to the total weight of the composition.

The make-up composition in accordance with a second aspect of the invention comprises a silicone polymer, in particular a glycerolated silicone polymer.

The term "silicone polymer" is intended to denote organosiloxanes.

The term "glycerolated silicone polymer" is intended to denote organosiloxanes carrying one or more glycerol group(s).

Silicone polymers in accordance with the silicone polymers of general formula (I) which can be used in the makeup compositions according to the present invention are described in detail in Patent Application EP 1 213 316.

In particular, the silicone polymers which can be used in the cosmetic compositions according to the present invention are represented by the following general formula (I'):

$$R^1_a\ R^2_b\ R^3_c\ SiO_{(4-a-b-c)/2} \qquad (I')$$

in which:

a) a, b and c are such that a varies from 1 to 2.5, and b and c, independently of one another, vary from 0.001 to 1.5,
b) $R^1$, identical or different, is chosen from:

- $C_1$ to $C_{30}$ alkyl radicals, if appropriate substituted by one or more fluorine atoms, amino groups and/or carboxyl groups,
- aryl or aralkyl radicals,
- radicals of general formula (II'):

$$-C_dH_2d\text{-}O\text{-}(C_2H_4O)e(C_3H_6O)_fR^4 \qquad (II')$$

in which:

- $R^4$ is a $C_1$ to $C_{30}$ hydrocarbon radical or an $R^5$-(CO)- radical with $R^5$ being a $C_1$ to $C_{30}$ hydrocarbon radical, and
- d, e and f are integers such that d varies from 0 to 15, and e and f, independently of one another, vary from 0 to 50, and

- their combinations,

c) $R^2$ is represented by the following general formula (III'):

$$-Q\text{-}O\text{-}X \qquad (III')$$

in which:

- Q is a divalent $C_2$ to $C_{20}$ hydrocarbon radical which can include at least one ether bond and/or at least one ester bond, and
- X is a polyhydroxylated hydrocarbon radical, in particular a polyhydroxy($C_1$-$C_6$)alkyl radical, such as $-[CH_2\text{-}CH(OH)\text{-}CH_2\text{-}O]_p\text{-}H$, with p between 1 and 10, preferably equal to 3.

d) $R^3$ is an organosiloxane group of general formula (IV'):

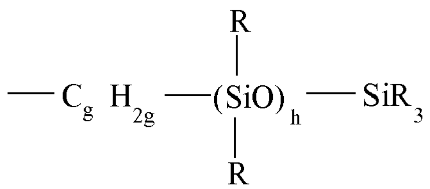

(IV')

with:

- each of the R radicals representing, independently of one another, a radical chosen from $C_1$ to $C_{30}$ alkyl radicals, if appropriate substituted by one or more fluorine atoms, and aryl and aralkyl radicals,
- g and h being integers such that g varies from 1 to 5 and h varies from 0 to 500.

When the R radicals represent a radical chosen from $C_1$ to $C_{30}$ alkyl radicals, if appropriate substituted by one or more fluorine atoms, aryl radicals and aralkyl radicals, they have the same meaning as the $R^1$ radical as defined above.
It should be noted that the $R^1$, $R^2$ and $R^3$ radicals of the silicone polymers of general formula (I') as defined above are distributed randomly or statistically, that is to say that they appear in the structure of the polymer without a predetermined order. Likewise, $R^1$, $R^2$, and $R^3$ can respectively represent radicals of different nature in a compound of general formula (I').
According to a specific embodiment,
in a):

- more particularly, a varies from 1.2 to 2.3 and, in particular, b and c, independently of one another, vary from 0.05 to 1,

in b):

- when $R^1$ is an alkyl radical, it can be a $C_1$ to $C_{30}$ alkyl radical, in particular a $C_1$ to $C_{25}$ alkyl radical, more particularly a $C_1$ to $C_{20}$ alkyl radical, in particular a $C_1$ to $C_{10}$ alkyl radical, and especially a $C_1$ to $C_6$ alkyl radical, and in particular a $C_1$ to $C_4$ alkyl radical. More particularly, it can be a methyl, ethyl, n- or isopropyl, n- or iso- or tert-butyl, pentyl, hexyl, heptyl, octyl, nonyl, decyl, undecyl or lauryl radical. It can also be a cycloalkyl radical, such as a cyclopropyl, a cyclobutyl, a cyclopentyl or a cyclohexyl. It can also be a linear or branched and monounsaturated or polyunsaturated alkyl radical. It can also be an alkyl radical substituted by one or more fluorine atoms, such as trifluoropropyl or heptadecafluorodecyl. It can also be an alkyl radical substituted by one or more amino groups, such as 2-aminoethyl, 3-aminopropyl or 3-[(2-aminoethyl)amino]propyl. It can also be an alkyl group substituted by one or more carboxyl groups, such as 3-carboxypropyl.
- $R^1$ can also be an aryl or aralkyl radical, such as the phenyl radical, the tolyl radical, the benzyl radical and the phenethyl radical,
- $R^1$ can also be an organic group represented by the general formula (II'):

$$-C_dH_{2d}-O-(C_2H_4O)_e(C_3H_6O)_fR^4 \qquad (II')$$

According to a specific embodiment, $R^1$ can be a hydroxylated radical or a radical resulting from the addition reaction of a saturated or unsaturated and a linear or branched alkenyl ether, in which d = 0 and thus of formula:

$$-O-(C_2H_4O)_e(C_3H_6O)_fR^4$$

In this case, when e and f equal zero, then $R^1$ is an alkoxy group having from 4 to 30 carbon atoms, for example a lower $C_4$ to $C_{10}$ alkoxy radical, such as butoxy or pentoxy, or a higher $C_{11}$ to $C_{30}$ alkoxy radical, such as oleoxy or stearoxy, namely, for example, cetyl alcohol, oleyl alcohol and stearyl alcohol, or a radical resulting from an acid or a fatty acid, such as acetic acid, lactic acid, butyric acid, oleic acid, stearic acid and behenic acid.
When e and f are greater than 1, then $R^1$ is a hydroxyl radical originating from the addition reaction of an alkylene oxide.
When e and f are equal to zero, it is particularly advantageous for d to be equal to 3, 5 or 11. In this case, $R^1$, according to the nature of the $R^4$ substituent, is an allyl ether, pentenyl ether or undecenyl ether radical or an allyl stearyl ether, pentenyl behenyl ether or undecenyl oleyl ether radical.
When e or f are other than zero, an alkoxy radical and an ester radical are present via a polyoxyalkylene group.
Whatever e and f, it is particularly advantageous for d to be within the range varying from 3 to 5.
According to one embodiment, the $R^1$ radical can be any one of the radicals defined above or a combination of two or more of these radicals.
Advantageously, $R^1$is an alkyl radical chosen from the methyl radical, the lauryl radical and their combinations.
Furthermore, when $R^1$ represents, in the same general formula (I), two or more radicals, for example a methyl radical and a lauryl radical, these radicals appear randomly in the structure and with a frequency which is specific to them.
Specifically, at least 50% of the $R^1$ radicals, in particular at least 70% of the $R^1$ radicals and more particularly 100% of the $R^1$ radicals, are methyl radicals.
in c):

- Q can in particular be a divalent hydrocarbon radical chosen from:
- $(CH_2)_2$-, $-(CH_2)_3$-, $-CH_2CH(CH_3)-CH_2$, $-(CH_2)_4$-, $-(CH_2)_5$-, $-(CH_2)_6$-, $-(CH_2)_7$-, $-(CH_2)_8$-, $-(CH_2)_9$-, $-(CH_2)_{10}$-, $-(CH_2)_{11}$-, $-(CH_2)_2-CH(CH_2CH_2CH_3)$-, $-CH_2-CH(CH_2CH_3)$-, $-(CH_2)_3-O-(CH_2)_2$-, $-(CH_2)_3-O-(CH_2)_2-O-(CH_2)_2$-, $-(CH_2)_3-O-CH_2CH(CH_3)$- and $-CH_2-CH(CH_3)-COO(CH_2)_2$-.

Advantageously, Q is a divalent radical chosen from $-(CH_2)_2$- and $-(CH_2)_3$-.
**[0178]** - X can in particular be a polyhydroxylated hydrocarbon radical comprising at least two hydroxyl residues and in particular a hydrocarbon group chosen from glycerylated derivatives and glycoside derivatives.
The glycerol residues can be compounds having the following formulae, in which Q has the same meaning as in the general formula (III'), and s and t are integers within the range varying from 1 to 20, in particular from 1 to 15, in particular from 1 to 10 and more particularly from 1 to 5.

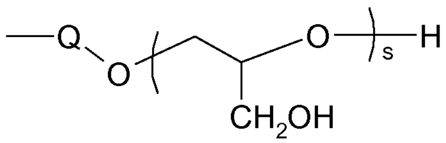

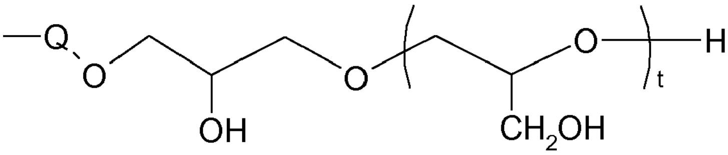

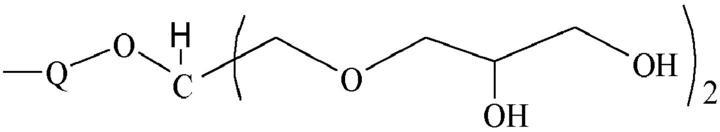

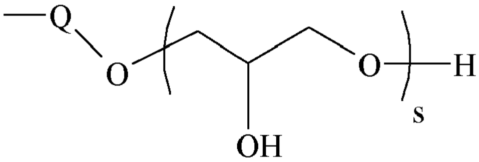

In the preceding formulae, one or more hydroxyl groups may be replaced by alkoxy groups or ester groups.

The glycoside radicals which are used in the general formula (III') can be of monosaccharide type, such as glycosyl, mannosyl, galactosyl, ribosyl, arabinosyl, xylosyl or fructosyl groups, of oligosaccharide type, such as maltosyl, cellobiosyl, lactosyl or maltotriosyl, or of polysaccharide type, such as cellulose or starch.

In particular, the glycoside groups are of monosaccharide or oligosaccharide type.

in d):

- each of the R radicals can represent in particular, independently of one another, a radical chosen from $C_1$ to $C_{20}$, more particularly $C_1$ to $C_{10}$, in particular $C_1$ to $C_6$, alkyl radicals, if appropriate substituted by one or more fluorine atoms. When the R radicals represent a radical chosen from the alkyl radicals as defined above, if appropriate substituted by one or more fluorine atoms, they have the same meaning as the $R^1$ radical as defined above.
- g, according to a specific embodiment, is equal to 2,
- h, according to a specific embodiment, is within the range varying from 1 to 50.

However, according to some of the specific aspects of the present invention, as defined above, the silicone polymer of general formula (I') is such that, when the $R^2$ radical is represented by the general formula (IIIA'):

$$-C_3H_6O[CH_2CH(OH)CH_2O]_nH \qquad \text{(IIIA')}$$

in which n varies from 1 to 5, then the $R^1$ radical is other than a $C_{12}$ alkyl radical.

According to a specific embodiment, the silicone polymer of general formula (I') suitable for the implementation of the present invention is such that:

- a varies from 1 to 1.4, and b and c, independently of one another, vary from 0.02 to 0.03, and
- $R^1$ is a $C_1$ to $C_{10}$, in particular $C_1$ to $C_6$ and more particularly $C_1$ to $C_4$ alkyl radical,
- $R^2$ is represented by the formula (IIIA'):

$$C_3H_6O[CH_2CH(OH)CH_2O]_n\,H \qquad \text{(IIIA')}$$

in which:

- n varies from 1 to 5, and
- $R^3$ is represented by the formula (IVA'):

$$-C_2H_4(CH_3)_2SiO[(CH_3)_2SiO]_m\ Si(CH_3)_3 \qquad \text{(IVA')}$$

in which:

- m varies from 3 to 9.

According to another specific embodiment, the silicone polymer of general formula (I') which can be used in the cosmetic compositions according to the present invention is such that:

- a varies from 1 to 1.4, and b and c, independently of one another, vary from 0.02 to 0.04,
- $R^1$ is a methyl radical,
- $R^2$ is represented by the formula (IIIA') in which n varies from 1 to 5, and
- $R^3$ is represented by the formula (IVA') in which m varies from 3 to 9.

Advantageously, the silicone polymer of general formula (I) used in the cosmetic composition in accordance with the invention may be chosen from polyglycerol-3 polydimethylsiloxyethyl dimethicone, lauryl polyglyceryl-3 polydimethylsiloxyethyl dimethicone and polyglyceryl-3 disiloxane dimethicone, the formulae of which are respectively:

- polyglyceryl-3 polydimethylsiloxyethyl dimethicone (formula (V')):

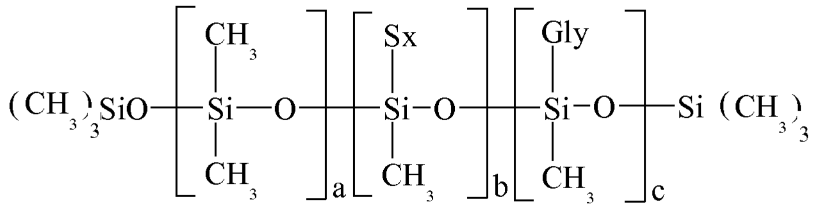

(V')

in which:

Sx: $-C_2H_4\ [(CH_3)_2SiO]_mSi(CH_3)_3$
Gly: $-C_3H_6O[CH_2\text{-}CH(OH)CH_2O]_nH$
with a = 1-1.4, b = 0.02-0.04, c = 0.02-0.04, m = 3-9 and n = 1-5,

- lauryl polyglyceryl-3 polydimethylsiloxyethyl dimethicone (formula (VI')):

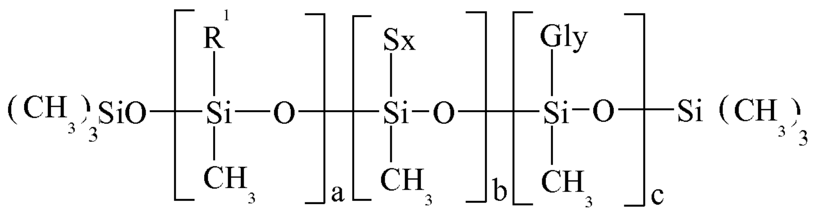

(VI')

in which Sx, Gly, a, b, c, m and n have the same meanings as above and $R_1$ is either a methyl radical or a lauryl radical,

- polyglyceryl-3 disiloxane dimethicone (formula (VII')):

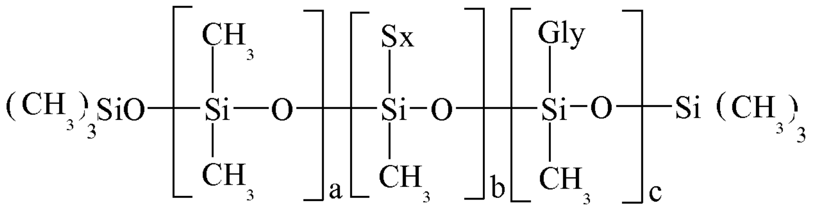

(VII')

in which Gly, a, b, c, m and n have the same meanings as above and

Sx: $-O(CH_3)_2SiO\text{-}Si(CH_3)_3$

According to a specific embodiment, the silicone polymer corresponds to the following formula (VIII'):

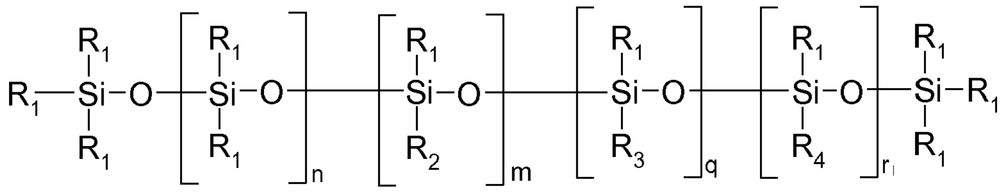

(VIII')

in which formula (VIII'):

- ○ $\mathbf{R_1}$, which are identical or different, represent:
  - i) a linear or branched $(C_1\text{-}C_{20})$alkyl group, in particular a linear or branched $C_1\text{-}C_6$ alkyl group, such as methyl, ethyl, propyl or butyl; or
  - ii) a hydroxyl group; preferably, $\mathbf{R_1}$ represents a methyl;
- ○ $\mathbf{R_2}$ represents the polysiloxane group -ALK-$[Si(R_1)_2\text{-}O]_{n'}\text{-}Si(R_1)_3$, with $R_1$ as defined above and ALK representing a divalent $(C_1\text{-}C_6)$alkylene group, such as methylene, ethylene or propylene, preferably ethylene;
- ○ $\mathbf{R_3}$ represents the -ALK'-O-$[CH_2\text{-}CH(OH)\text{-}CH_2\text{-}O]_p$-H group, with p between 1 and 10 inclusive; preferably, p is equal to 3; and ALK' representing a divalent $(C_1\text{-}C_6)$alkylene group, such as methylene, ethylene or propylene, preferably propylene;
- ○ $\mathbf{R_4}$ represents a $(C_1\text{-}C_{20})$alkyl group which is linear or branched, in particular one comprising from 8 to 16 carbon atoms, in particular one which is linear; preferably, one comprising 12 carbon atoms, such as lauryl;
- ○ **m** and **r**, which are identical or different, are an integer inclusively between 0 and 150, preferably 20 and 100;
- ○ **n, n'** and **q**, which are identical or different, are an integer inclusively between 1 and 300, preferably 1 and 100.

According to a preferred embodiment, the silicone polymer corresponds to the following formula (IX') or (X'):

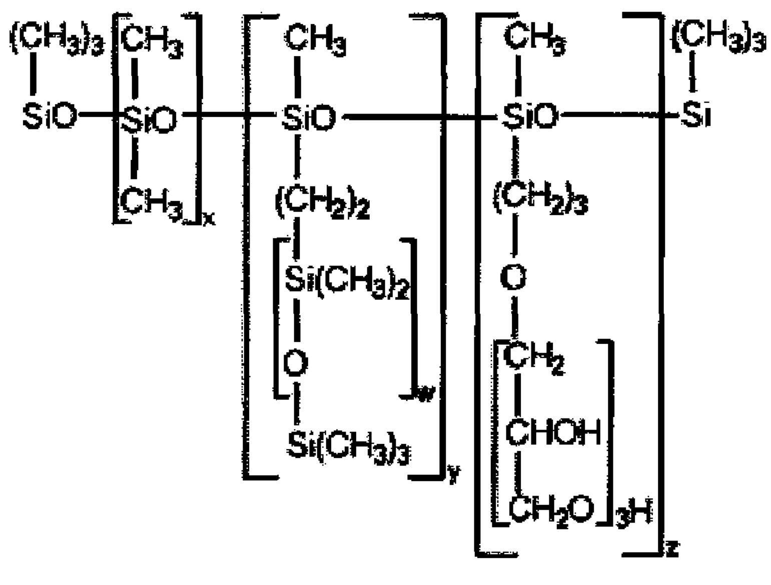

(IX')

in which formula (IX') x, y and z, which are identical or different, are an integer inclusively between 1 and 25, preferably between 1 and 20, better still between 1 and 10, and w is an integer between 1 and 25, preferably between 1 and 20, better still between 1 and 10. The molecular weight of such a compound can, for example, be approximately 10 000 g/mol. Preferably, x, y, z and w are each equal to 9.

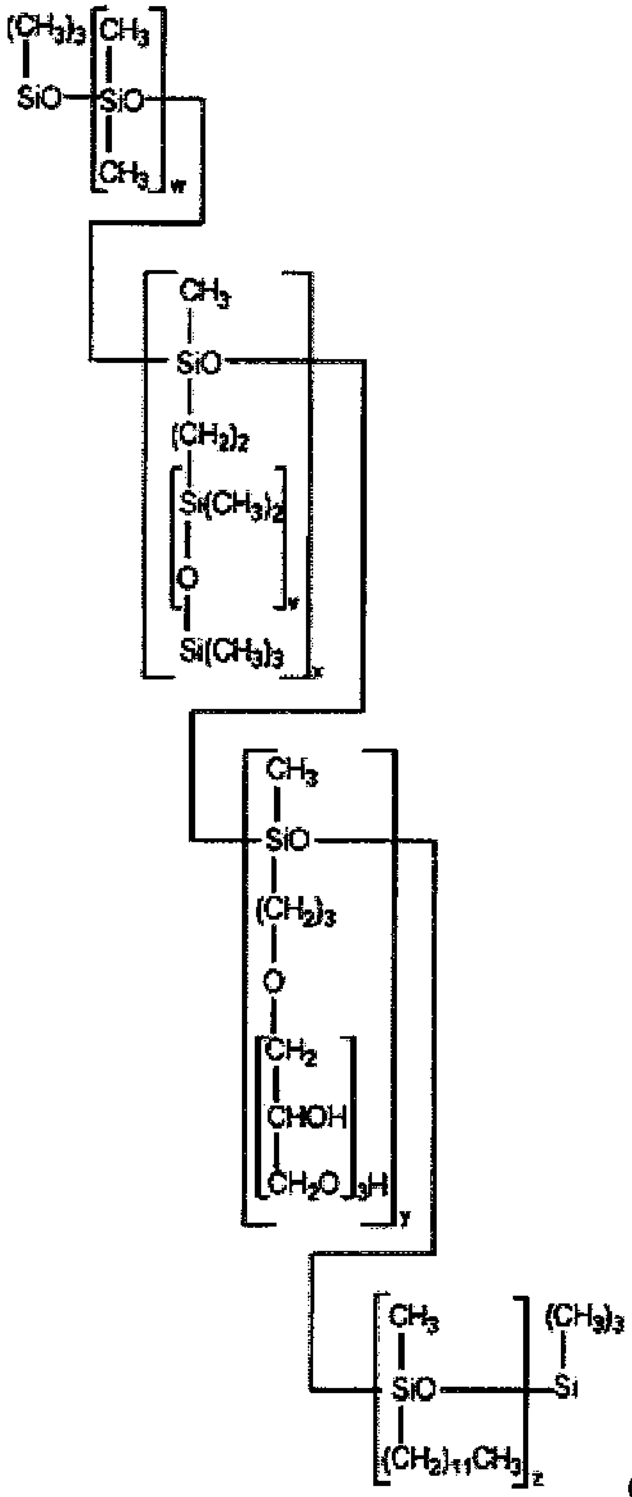

(X')

in which formula (X') x, y and z, which are identical or different, are an integer inclusively between 1 and 25, preferably between 1 and 20 and better still between 1 and 10 and w is an integer between 1 and 25, preferably between 1 and 20 and better still between 1 and 10.

According to this second aspect, the present invention comprises at least one silicone polymer of general formula (I') or one mixture of silicone polymers, at least one of which corresponds to this general formula (I').

Preferably, the silicone polymer is present in the composition according to the invention in a content ranging from 0.1 to 40% by weight, particularly from 0.5 to 35% by weight and for example from 2 to 25% by weight, better still from 5 to 20% by weight, indeed even from 8 to 16% by weight, with respect to the total weight of the composition.

The silicone polymer of general formula (I') is in particular employed in the present invention in a free form.

Within the meaning of the present invention, the term "free form" is intended to denote a form of the silicone polymer of general formula (I') in which the polymer is not employed in a form combined with or adsorbed on another material, as described, for example, in the documents EP 1 416 016 and EP 1 424 373, where it is present in the form of a coating of a powder or of a colouring agent in order to block the surface activity of the particles constituting the corresponding powder.
According to a specific embodiment, the silicone polymer of general formula (I') is advantageously chosen from the polymers sold by Shin-Etsu under the references KF6100®, KF6104® and KF6105®.
According to yet another embodiment, the polymer sold under the reference KF6104®, sold by Shin-Etsu, is particularly suitable for the make-up removal of cosmetic compositions comprising a compound conferring, on the said composition, a dmax threading nature of greater than or equal to 5 mm.
According to yet another embodiment, the compound denoted under the reference KF6105®, sold by Shin-Etsu, is particularly suitable for the make-up removal of cosmetic compositions comprising a compound or a mixture of compounds conferring, on the said composition, a dmax threading nature of greater than or equal to 5 mm.
The present invention comprises, according to a third aspect of the invention, at least one phenylated silicone oil or a mixture of silicone oils, at least one of which is phenylated.
The term "phenylated silicone oil" denotes an organopolysiloxane comprising at least one phenyl group.
The phenylated silicone oil is preferably nonvolatile.
The term "nonvolatile oil" is understood to mean an oil which remains on the skin or the keratinous fibre, more generally on the keratinous substance, at ambient temperature and atmospheric pressure, for at least several hours and which has in particular a vapour pressure of less than $10^{-3}$ mmHg (0.13 Pa). A nonvolatile oil can also be defined as having a rate of evaporation such that, under the conditions defined above, the amount evaporated after 30 minutes is less than 0.07 mg/cm$^2$.
Preferably, the molecular weight of the phenylated silicone oil is between 500 and 100 000 g/mol.
Preferably, the phenylated silicone oil is chosen from the group consisting of the compounds of following formulae (I"), (II"), (III"), (IV"), (V"), (VI") and (VII"):

- compounds of formula (I"):

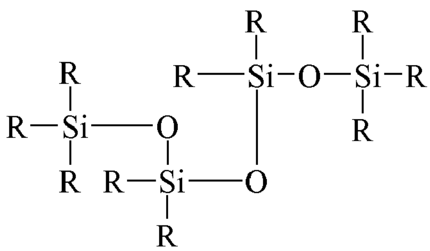

(I'')

- compounds of formula (II"):

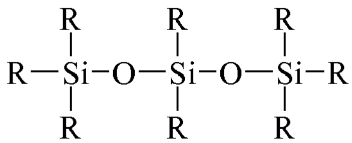

(II'')

the formulae (I") and (II") being such that the R groups independently represent a methyl group or a phenyl group and that at least three of the R radicals are phenyl groups, indeed even at least four, in particular at least five. Use may be made, for example, of trimethylpentaphenyltrisiloxane (or 1,3,5-trimethyl-1,1,3,5,5-pentaphenyltrisiloxane), sold under the reference PH-1555 HRI by Dow Coming.

- compounds of formula (III"):

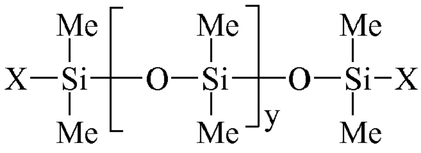

(III'')

in which X represents a $-CH_2-CH(CH_3)(Ph)$ group, Me represents a methyl group and Ph represents a phenyl group, and y varies between 1 and 10 000,

- compounds of formula (IV"):

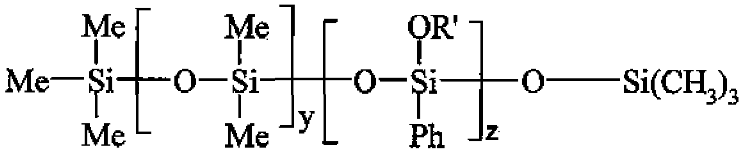

(IV'')

in which Me is methyl and Ph is phenyl, OR' represents an $-OSiMe_3$ group, y varies between 1 and 1000 and z varies between 1 and 1000, so that the compound (IV") is a nonvolatile oil. Use may be made, for example, of trimethylsiloxyphenyl dimethicone, sold in particular under the reference BELSIL PDM 1000 by Wacker.

- compounds of formula (V"):

$$[R(CH_3)_2\text{-}SiO_{1/2}]_x[SiO_{4/2}]_y \qquad (V'')$$

in which R represents a phenylpropyl group, and x and y vary, independently of one another, between 1 and 10 000, so that x+y is sufficiently high for the compound (V") to be a nonvolatile oil. Use may be made, for example, of phenylpropyldimethyl-siloxysilicate, sold under the reference SilShine 151 by GE Silicones.

- compounds of formula (VI"):

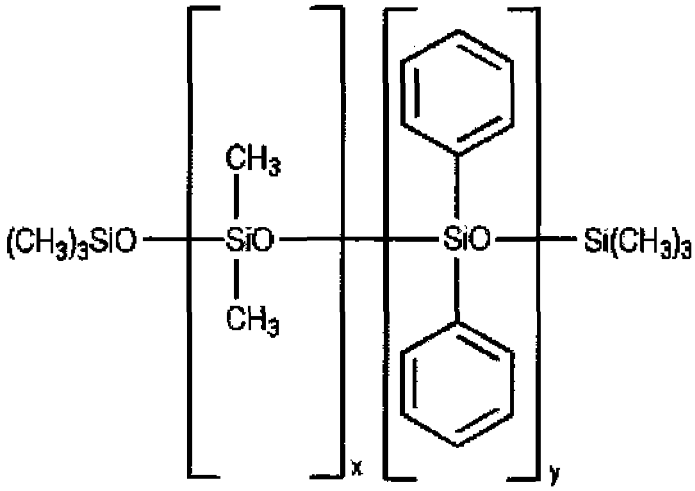

(VI'')

in which x and y, which are identical or different, and in particular x and y being an integer varying for both from 1 to 50, better still from 1 to 20 and more specifically from 1 to 10. Use may be made, for example, of a phenylated silicone oil sold by Shin-Etsu under the reference KF54® (400 cSt). The molecular weight of such a compound can, for example, be approximately 3000 g/mol. Preferably, x and y are both equal to 10.

- compounds of formula (VII"):

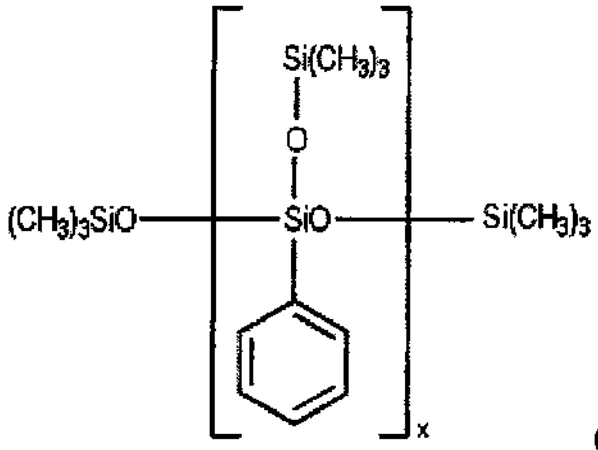

(VII")

in which x is an integer varying from 1 to 50, better still from 1 to 20 and more specifically from 1 to 10. Use may be made, for example, of a phenylated silicone oil sold by PCR under the reference 15M30® (500 cSt). The molecular weight of such a compound can, for example, be approximately 372 g/mol. Preferably, x is equal to 1.

According to a specific embodiment, the composition according to the invention comprises at least one phenylated silicone oil of the formula (IV"), (V"), (VI") or (VII") defined above.

According to a specific embodiment, the composition according to the invention comprises at least one phenylated silicone oil of formula (IV") or (VI").

The phenylated silicone oil of formulae (II"), (III"), (IV"), (V"), [VI"] and (VII") preferably exhibits a refractive index of greater than 1.4, in particular of less than 1.6.

The phenylated silicone oils have a viscosity advantageously chosen within the range extending from 5 to 20 000 $mm^2/s$ at 25°C, preferably from 10 to 10 000 $mm^2/s$, better still from 10 to 5000 $mm^2/s$ and even better still from 10 to 1000 $mm^2/s$.

Such phenylated silicone oils exhibiting, at 25°C, a viscosity of greater than or equal to 400 cSt are particularly suitable for the make-up removal of cosmetic compositions comprising a compound or a mixture of compounds conferring, on the said composition, a dmax threading nature of greater than or equal to 5 mm.

The method for measuring the viscosity used in the invention in order to characterize the silicone oils according to the invention can be "kinematic viscosity at 25°C raw product CID-012-01" or also "Viscosity Ubbelohde DIN 51562-1 PV04001 25°C".

Preferably, the phenylated silicone oil is present in the composition according to the invention in a content ranging from 0.1 to 40% by weight, particularly from 0.5 to 35% by weight, for example from 2 to 25% by weight, better still from 5 to 20% by weight, indeed even from 8 to 15% by weight, with respect to the total weight of the composition.

## AQUEOUS PHASE

The composition according to the invention can comprise an aqueous medium, constituting an aqueous phase, which can form the continuous phase of the composition.

In a preferred embodiment, the composition according to the invention includes less than 10% of water by weight with respect to the total weight of the composition, preferably less than 8% by weight.

In a variant, the aqueous phase of the composition according to the invention is advantageously a continuous aqueous phase.

Composition comprising a "continuous" aqueous phase is understood to mean that the composition exhibits a conductivity, measured at 25°C, of greater than 23 μS/cm (microSiemens/cm), the conductivity being measured, for example, using an MPC227 conductivity meter from Mettler Toledo and an Inlab730 conductivity measurement cell. The measurement cell is immersed in the composition, so as to remove the air bubbles capable of being formed between the 2 electrodes of the cell. The conductivity is read as soon as the value of the conductivity meter has stabilized. A mean is taken over at least 3 successive measurements.

The aqueous phase can be composed essentially of water; it can also comprise a mixture of water and of water-miscible solvent (miscibility in water of greater than 50% by weight at 25°C), such as lower monoalcohols having from 1 to 5 carbon atoms, such as ethanol or isopropanol, glycols having from 2 to 8 carbon atoms, such as propylene glycol, ethylene glycol, 1,3-butylene glycol or dipropylene glycol, $C_3$-$C_4$ ketones, $C_2$-$C_4$ aldehydes and their mixtures.

The aqueous phase (water and optionally the water-miscible solvent) can be present in a content ranging from 1 to 95% by weight, with respect to the total weight of the composition, preferably ranging from 3 to 80% by weight and

preferentially ranging from 5 to 60% by weight.

According to a specific and preferred embodiment, the composition comprises less than 10% by weight of water and water-miscible solvent, with respect to the total weight of the composition, in particular less than 5% by weight, indeed even less than 2% by weight. According to a specific embodiment, the composition is anhydrous. In such cases, the cosmetic composition can be provided in the form of a stick or piece of product. This stick can be solid at ambient temperature (20°C), that is to say that it retains the shape which was given to it on manufacture and does not flow under the effect of gravity. Such a piece of product can, for example, exhibit a hardness in shearing at 20°C of greater than 375 g/m, indeed even of greater than 5000 g/m or of greater than 10 000 g/m, or also of greater than 12 000 g/m and in particular of between 375 g/m and 15 000 g/m, according to the method defined above. A greater hardness can improve the mechanical strength of the product and can facilitate the packaging and use thereof.

<u>**Emulsifying system**</u>

The compositions according to the invention can comprise an emulsifying system comprising surface-active agents present in particular in a proportion ranging from 0.1 to 20% and better still from 0.3 to 15% by weight, with respect to the total weight of the composition.

Use is generally made of an emulsifier appropriately chosen in order to obtain an oil-in-water emulsion. Use may in particular be made of an emulsifier having, at 25°C, an HLB (hydrophilic-lipophilic balance) balance within the meaning of Griffin of greater than or equal to 8.

The HLB value according to Griffin is defined in J. Soc. Cosm. Chem., 1954 (volume 5), pages 249-256.

These surface-active agents can be chosen from nonionic, anionic, cationic or amphoteric surface-active agents or also surface-active emulsifiers. Reference may be made to the document "Encyclopedia of Chemical Technology, Kirk-Othmer", volume 22, pp. 333-432, 3rd edition, 1979, Wiley, for the definition of the properties and functions (emulsifying) of surfactants, in particular pp. 347-377 of this reference for the anionic, amphoteric and nonionic surfactants.

The surfactants preferentially used in the composition according to the invention are chosen from:

a) nonionic surface-active agents with an HLB of greater than or equal to 8 at 25°C, used alone or as a mixture; mention may in particular be made of:

- oxyethylenated and/or oxypropylenated ethers (which can comprise from 1 to 150 oxyethylene and/or oxypropylene groups) of glycerol;
- oxyethylenated and/or oxypropylenated ethers (which can comprise from 1 to 150 oxyethylene and/or oxypropylene groups) of fatty alcohols (in particular of $C_8$-$C_{24}$ and preferably $C_{12}$-$C_{18}$ alcohols), such as the oxyethylenated ether of stearyl alcohol comprising 20 oxyethylene groups (CTFA name "Steareth-20"), such as Brij 78, sold by Uniqema, the oxyethylenated ether of cetearyl alcohol comprising 30 oxyethylene groups (CTFA name "Ceteareth-30") and the oxyethylenated ether of the mixture of $C_{12}$-$C_{15}$ fatty alcohols comprising 7 oxyethylene groups (CTFA name "C12-15 Pareth-7") sold under the name Neodol 25-7® by Shell Chemicals,
- esters of fatty acid (in particular of $C_8$-$C_{24}$ and preferably $C_{16}$-$C_{22}$ acid) and of polyethylene glycol (which can comprise from 1 to 150 ethylene glycol units), such as PEG-50 stearate and PEG-40 monostearate, sold under the name Myrj 52P® by ICI Uniqema,
- esters of fatty acid (in particular of $C_8$-$C_{24}$ and preferably $C_{16}$-$C_{22}$ acid) and of the oxyethylenated and/or oxypropylenated glycerol ethers (which can comprise from 1 to 150 oxyethylene and/or oxypropylene groups), such as PEG-200 glyceryl monostearate, sold under the name Simulsol 220 TM® by SEPPIC; polyethoxylated glyceryl stearate comprising 30 ethylene oxide groups, such as the product Tagat S® sold by Goldschmidt, polyethoxylated glyceryl oleate comprising 30 ethylene oxide groups, such as the product Tagat O® sold by Goldschmidt, polyethoxylated glyceryl cocoate comprising 30 ethylene oxide groups, such as the product Varionic LI 13® sold by Sherex, polyethoxylated glyceryl isostearate comprising 30 ethylene oxide groups, such as the product Tagat L® sold by Goldschmidt, and polyethoxylated glyceryl laurate comprising 30 ethylene oxide groups, such as the product Tagat I® from Goldschmidt,
- esters of fatty acid (in particular of $C_8$-$C_{24}$ and preferably $C_{16}$-$C_{22}$ acid) and of the oxyethylenated and/or oxypropylenated sorbitol ethers (which can comprise from 1 to 150 oxyethylene and/or oxypropylene groups), such as polysorbate 60, sold under the name Tween 60® by Uniqema,
- dimethicone copolyol, such as that sold under the name Q2-5220® by Dow Coming,
- dimethicone copolyol benzoate (Finsolv SLB 101® and 201® from Fintex),
- copolymers of propylene oxide and of ethylene oxide, also known as EO/PO polycondensates,
- and their mixtures.

The EO/PO polycondensates are more particularly copolymers consisting of polyethylene glycol and polypropylene glycol blocks, such as, for example, polyethylene glycol/polypropylene glycol/polyethylene glycol triblock polycondensates. These triblock polycondensates have, for example, the following chemical structure:

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

in which formula a ranges from 2 to 120 and b ranges from 1 to 100.

The EO/PO polycondensate preferably has a weight-average molecular weight ranging from 1000 to 15 000 and better still ranging from 2000 to 13 000. Advantageously, the said EO/PO polycondensate has a cloud point, at 10 g/l in distilled water, of greater than or equal to 20°C, preferably of greater than or equal to 60°C. The cloud point is measured according to the standard ISO 1065. Mention may be made, as EO/PO polycondensate which can be used according to the invention, of the polyethylene glycol/polypropylene glycol/polyethylene glycol triblock polycondensates sold under the Synperonic® names, such as Synperonic PE/L44® and Synperonic PE/F127®, by ICI,

b) nonionic surface-active agents with an HLB of less than 8 at 25°C, optionally in combination with one or more nonionic surface-active agents with an HLB of greater than 8 at 25°C, such as mentioned above, such as:

esters and ethers of monosaccharides, such as sucrose stearate, sucrose cocoate, sorbitan stearate and their mixtures, such as Arlatone 2121®, sold by ICI, or Span 65V, from Uniqema;

esters of fatty acids (in particular of $C_8$-$C_{24}$ and preferably $C_{16}$-$C_{22}$ acid) and of polyol, in particular of glycerol or of sorbitol, such as glyceryl stearate, such as the product sold under the name Tegin M® by Goldschmidt, glyceryl laurate, such as the product sold under the name Imwitor 312® by Hüls, polyglyceryl-2 stearate, sorbitan tristearate or glyceryl ricinoleate;

oxyethylenated and/or oxypropylenated ethers, such as the oxyethylenated ether of stearyl alcohol comprising 2 oxyethylene groups (CTFA name "Steareth-2"), such as Brij 72, sold by Uniqema;

the cyclomethicone/dimethicone copolyol mixture sold under the name Q2-3225C® by Dow Coming,

c) anionic surfactants, such as:

salts of polyoxyethylenated fatty acids, in particular those deriving from amines or alkali metal salts, and their mixtures;

phosphoric esters and their salts, such as "DEA oleth-10 phosphate" (Crodafos N 10N from Croda) or monopotassium monocetyl phosphate (Amphisol K from Givaudan or Arlatone MAP 160K from Uniqema);

sulphosuccinates, such as "Disodium PEG-5 citrate lauryl sulphosuccinate" and "Disodium ricinoleamido MEA sulphosuccinate";

alkyl ether sulphates, such as sodium lauryl ether sulphate;

isethionates;

acylglutamates, such as "Disodium hydrogenated tallow glutamate" (Amisoft HS-21 R®, sold by Ajinomoto), and their mixtures.

Mention may in particular be made, by way of representation of cationic surfactants, of:

- alkylimidazolidiniums, such as isostearylethylimidonium ethosulphate,
- ammonium salts, such as N,N,N-trimethyl-1-docosanaminium chloride (behentrimonium chloride).

The compositions according to the invention can also comprise one or more amphoteric surfactants, such as N-acylamino acids, for example N-alkylamino acetates and disodium cocoamphodiacetate, and amine oxides, such as stearamine oxide, or also silicone surfactants, such as dimethicone copolyol phosphates, such as that sold under the name Pecosil PS 100® by Phoenix Chemical.

**Water-soluble gelling agent**

The composition according to the invention can comprise a water-soluble gelling agent.

The water-soluble gelling agents which can be used in the compositions according to the invention can be chosen from:

homo- or copolymers of acrylic acid or methacrylic acid or their salts and their esters and in particular the products sold under the names Versicol F® or Versicol K® by Allied Colloid, Ultrahold 8® by Ciba-Geigy, poly(acrylic acid)s of Synthalen K type,

copolymers of acrylic acid and of acrylamide, sold in the form of their sodium salts under the Reten® names by Hercules, poly(sodium methacrylate), sold under the name Darvan No. 7® by Vanderbilt, or sodium salts of poly(hydroxycarboxylic acid)s, sold under the name Hydagen F® by Henkel,
copolymers of poly(acrylic acid)s and of alkyl acrylates of Pemulen type,
AMPS (poly(acrylamidomethylpropanesulphonic acid) partially neutralized with aqueous ammonia and highly crosslinked), sold by Clariant,
AMPS/acrylamide copolymers of Sepigel® or Simulgel® type, sold by SEPPIC, and
copolymers of AMPS and of alkyl methacrylates which are polyoxyethylenated (crosslinked or noncrosslinked),
proteins, such as proteins of plant origin, such as wheat or soya proteins; or proteins of animal origin, such as keratins, for example keratin hydrolysates and sulphonic keratins;
cellulose polymers, such as hydroxyethylcellulose, hydroxypropylcellulose, methylcellulose, ethylhydroxyethylcellulose, carboxymethylcellulose and quaternized cellulose derivatives;
acrylic polymers or copolymers, such as polyacrylates or polymethacrylates;
vinyl polymers, such as polyvinylpyrrolidones, copolymers of methyl vinyl ether and of malic anhydride, the copolymer of vinyl acetate and of crotonic acid, copolymers of vinylpyrrolidone and of vinyl acetate, copolymers of vinylpyrrolidone and of caprolactam, or poly(vinyl alcohol);
optionally modified polymers of natural origin, such as:

gums arabic, guar gum, xanthan derivatives or karaya gum;
alginates and carrageenans;
glycoaminoglycans, hyaluronic acid and its derivatives;
shellac resin, gum sandarac, dammars, elemis or copals;
deoxyribonucleic acid;
mucopolysaccharides, such as chondroitin sulphates,
and their mixtures.

Some of these water-soluble gelling agents can also act as film-forming polymers.

According to a preferred embodiment, the composition comprises at least one AMPS/acrylamide copolymer.

The water-soluble gelling polymer can be present in the composition according to the invention in a content of dry matter ranging from 0.01 to 60% by weight, preferably from 0.5 to 40% by weight, better still from 1 to 30% by weight, indeed even from 5 to 20% by weight, with respect to the total weight of the composition.

## FATTY PHASE

The composition according to the invention can comprise a fatty phase comprising at least one oil and/or at least one wax and/or at least one lipophilic film-forming polymer and/or one fatty-phase rheological agent. In particular, this composition can be a continuous liquid fatty phase.

The term "liquid fatty phase" is understood to mean, within the meaning of the invention, a phase composed of one or more nonaqueous fatty substances which are liquid at ambient temperature (25°C) and atmospheric pressure (760 mmHg), also known as organic oils or solvents, and which are compatible with one another.

The liquid fatty phase represents from 10 to 98% by weight of the total weight of the composition, preferably from 20 to 85% by weight and better still from 40 to 80% by weight. The make-up composition can, for example, comprise a liquid fatty phase present at a content of greater than or equal to 40% by weight, with respect to the total weight of the composition, indeed even 60% by weight or also 80% by weight or even 95% by weight, with respect to the total weight of the composition.

The term "composition comprising a continuous liquid fatty phase" is understood to mean that the composition exhibits a conductivity, measured at 25°C, of less than 23 μS/cm (microSiemens/cm), the conductivity being measured, for example, using an MPC227 conductivity meter from Mettler Toledo and an Inlab730 conductivity measurement cell. The measurement cell is immersed in the composition, so as to remove the air bubbles liable to be formed between the 2 electrodes of the cell. The conductivity is read as soon as the value of the conductivity meter has stabilized. A mean is determined over at least 3 successive measurements.

### *Oils*

The term "oil" is understood to mean a fatty substance which is liquid at ambient temperature and atmospheric pressure.

Volatile oil

The composition according to the invention can comprise at least one volatile oil.

The term "volatile oil" is understood to mean an oil (or a nonaqueous medium) capable of evaporating on contact with the skin in less than one hour at ambient temperature and atmospheric pressure. The volatile oil is a volatile cosmetic oil which is liquid at ambient temperature and which has in particular a nonzero vapour pressure at ambient temperature and atmospheric pressure, especially having a vapour pressure ranging from 0.13 Pa to 40 000 Pa ($10^{-3}$ to 300 mmHg), preferably ranging from 1.3 Pa to 13 000 Pa (0.01 to 100 mmHg) and preferentially ranging from 1.3 Pa to 1300 Pa (0.01 to 10 mmHg).

This volatile oil can be a hydrocarbon oil.

The volatile hydrocarbon oil can be chosen from hydrocarbon oils having from 7 to 16 carbon atoms. The volatile hydrocarbon oil can be present in the composition according to the invention in a content ranging from 0.1% to 90% by weight, with respect to the total weight of the composition, preferably ranging from 1% to 70% by weight and preferentially ranging from 5% to 70% by weight, indeed even from 5% to 50% by weight.

The composition according to the invention can comprise one or more volatile branched alkane(s). The term "one or more volatile branched alkane(s)" is understood to mean without distinction "one or more volatile branched alkane(s) oil".

Mention may in particuar be made, as volatile hydrocarbon oil having from 7 to 16 carbon atoms, of branched $C_8$-$C_{16}$ alkanes, such as $C_8$-$C_{16}$ isoalkanes (also known as isoparaffins), isododecane, isodecane, isohexadecane and, for example, the oils sold under the Isopar or Permethyl tradenames, branched $C_8$-$C_{16}$ esters, such as isohexyl neopentanoate, and their mixtures. Preferably, the volatile hydrocarbon oil having from 8 to 16 carbon atoms is chosen from isododecane, isodecane, isohexadecane and their mixtures, and is in particular isododecane.

The composition according to the invention can comprise one or more volatile linear alkane(s). The term "one or more volatile linear alkane(s)" is understood to mean without distinction "one or more volatile linear alkane(s) oil".

A volatile linear alkane suitable for the invention is liquid at ambient temperature (approximately 25°C) and at atmospheric pressure (760 mmHg).

The term "volatile linear alkane" suitable for the invention is understood to mean a cosmetic linear alkane which is capable of evaporating on contact with the skin in less than one hour at ambient temperature (25°C) and atmospheric pressure (760 mmHg, that is to say 101 325 Pa), which is liquid at ambient temperature and which has in particular a rate of evaporation ranging from 0.01 to 15 mg/cm$^2$/min at ambient temperature (25°C) and atmospheric pressure (760 mmHg).

Preferably, the "volatile linear alkanes" suitable for the invention exhibit a rate of evaporation ranging from 0.01 to 3.5 mg/cm$^2$/min at ambient temperature (25°C) and atmospheric pressure (760 mmHg).

Preferably, the "volatile linear alkanes" suitable for the invention exhibit a rate of evaporation ranging from 0.01 to 1.5 mg/cm$^2$/min at ambient temperature (25°C) and atmospheric pressure (760 mmHg).

More preferably, the "volatile linear alkanes" suitable for the invention exhibit a rate of evaporation ranging from 0.01 to 0.8 mg/cm$^2$/min, at ambient temperature (25°C) and atmospheric pressure (760 mmHg).

More preferably, the "volatile linear alkanes" suitable for the invention exhibit a rate of evaporation ranging from 0.01 to 0.3 mg/cm$^2$/min, at ambient temperature (25°C) and atmospheric pressure (760 mmHg).

More preferably, the "volatile linear alkanes" suitable for the invention exhibit a rate of evaporation ranging from 0.01 to 0.12 mg/cm$^2$/min at ambient temperature (25°C) and atmospheric pressure (760 mmHg).

The rate of evaporation of a volatile alkane in accordance with the invention (and more generally of a volatile solvent) can be evaluated in particular by means of the protocol described in WO 06/013413 and more particularly by means of the protocol described below.

15 g of volatile hydrocarbon solvent are introduced into a crystallizing dish (diameter: 7 cm) placed on a balance located in a chamber with a capacity of approximately 0.3 m$^3$ regulated with regard to temperature (25°C) and hygrometry (relative humidity 50%).

The liquid is allowed to freely evaporate, without being stirred, ventilation being provided by a fan (Papst-Motoren, reference 8550 N, rotating at 2700 revolutions/minute) arranged in the vertical position above the crystallizing dish containing the volatile hydrocarbon solvent, the blades being directed towards the crystallizing dish, at a distance of 20 cm with respect to the bottom of the crystallizing dish.

The weight of volatile hydrocarbon solvent remaining in the crystallizing dish is measured at regular time intervals.

The evaporation profile of the solvent is then obtained by plotting the curve of the amount of product evaporated (in mg/cm$^2$) as a function of the time (in min).

The rate of evaporation, which corresponds to the tangent at the origin of the curve obtained, is then calculated. The rates of evaporation are expressed in mg of volatile solvent evaporated per unit of surface area (cm$^2$) and per unit of time (minute).

According to a preferred embodiment, the "volatile linear alkanes" suitable for the invention have a nonzero vapour pressure (also known as saturated vapour pressure) at ambient temperature, in particular a vapour pressure ranging from 0.3 Pa to 6000 Pa.

Preferably, the "volatile linear alkanes" suitable for the invention have a vapour pressure ranging from 0.3 to 2000 Pa, at ambient temperature (25°C).

Preferably, the "volatile linear alkanes" suitable for the invention have a vapour pressure ranging from 0.3 to 1000 Pa at ambient temperature (25°C).

More preferably, the "volatile linear alkanes" suitable for the invention have a vapour pressure ranging from 0.4 to 600 Pa at ambient temperature (25°C).

Preferably, the "volatile linear alkanes" suitable for the invention have a vapour pressure ranging from 1 to 200 Pa at ambient temperature (25°C).

More preferably, the "volatile linear alkanes" suitable for the invention have a vapour pressure ranging from 3 to 60 Pa at ambient temperature (25°C).

According to one embodiment, a volatile linear alkane suitable for the invention can exhibit a flash point within the range varying from 30 to 120°C and more particularly from 40 to 100°C. The flash point is in particular measured according to Standard ISO 3679.

According to one embodiment, an alkane suitable for the invention can be a volatile linear alkane comprising from 7 to 14 carbon atoms.

Preferably, the "volatile linear alkanes" suitable for the invention comprise from 8 to 14 carbon atoms.

Preferably, the "volatile linear alkanes" suitable for the invention comprise from 9 to 14 carbon atoms.

Preferably, the "volatile linear alkanes" suitable for the invention comprise from 10 to 14 carbon atoms.

Preferably, the "volatile linear alkanes" suitable for the invention comprise from 11 to 14 carbon atoms.

According to an advantageous embodiment, the "volatile linear alkanes" suitable for the invention exhibit a rate of evaporation, as defined above, ranging from 0.01 to 3.5 mg/cm$^2$/min at ambient temperature (25°C) and atmospheric pressure (760 mmHg) and comprise from 8 to 14 carbon atoms.

A volatile linear alkane suitable for the invention can advantageously be of vegetable origin.

Preferably, the volatile linear alkane or the mixture of volatile linear alkanes present in the composition according to the invention comprises at least a $^{14}C$ isotope of carbon (carbon-14); in particular, the $^{14}C$ isotope can be present in a $^{14}C/^{12}C$ ratio of greater than or equal to $1 \times 10^{-16}$, preferably of greater than or equal to $1 \times 10^{-15}$, more preferably of greater than or equal to $7.5 \times 10^{-14}$ and better still of greater than or equal to $1.5 \times 10^{-13}$. Preferably, the $^{14}C/^{12}C$ ratio ranges from $6 \times 10^{-13}$ to $1.2 \times 10^{-12}$.

The amount of $^{14}C$ isotopes in the volatile linear alkane or the mixture of volatile linear alkanes can be determined by methods known to a person skilled in the art, such as the Libby counting method, liquid scintillation spectrometry or accelerator mass spectrometry.

Such an alkane can be obtained, directly or in several stages, from a vegetable starting material, such as an oil, a butter, a wax, and the like.

Mention may be made, as example of alkanes suitable for the invention, of the alkanes described in the patent applications from Cognis, WO 2007/068371 or WO2008/155059 (mixtures of distinct alkanes differing by at least one carbon). These alkanes are obtained from fatty alcohols, themselves obtained from coconut or palm oil.

Mention may be made, as example of linear alkanes suitable for the invention, of n-heptane($C_7$), n-octane ($C_8$), n-nonane ($C_9$), n-decane ($C_{10}$), n-undecane ($C_{11}$), n-dodecane ($C_{12}$), n-tridecane ($C_{13}$), n-tetradecane ($C_{14}$) and their mixtures. According to a specific embodiment, the volatile linear alkane is chosen from n-nonane, n-undecane, n-dodecane, n-tridecane, n-tetradecane and their mixtures.

According to a preferred form, mention may be made of the mixtures of n-undecane ($C_{11}$) and of n-tridecane ($C_{13}$) obtained in Examples 1 and 2 of Application WO2008/155059 of Cognis.

Mention may also be made of the n-dodecane ($C_{12}$) and the n-tetradecane ($C_{14}$) sold by Sasol under the references Parafol 12-97 and Parafol 14-97 respectively, and their mixtures.

The volatile linear alkane can be used alone.

Alternatively or preferentially, use may be made of a mixture of at least two distinct volatile linear alkanes differing from one another by a carbon number n of at least 1, in particular differing from one another by a carbon number of 1 or 2.

According to a first embodiment, use is made of a mixture of at least two distinct volatile linear alkanes comprising from 10 to 14 carbon atoms and differing from one another by a carbon number of at least 1. Mention may be made, as examples, in particular of the $C_{10}/C_{11}$, $C_{11}/C_{12}$ or $C_{12}/C_{13}$ mixtures of volatile linear alkanes.

According to another embodiment, use is made of a mixture of at least two distinct volatile linear alkanes comprising from 10 to 14 carbon atoms and differing from one another by a carbon number of at least 2. Mention may be made, as examples, in particular of the $C_{10}/C_{12}$ or $C_{12}/C_{14}$ mixtures of volatile linear alkanes, for an even carbon number n, and the $C_{11}/C_{13}$ mixture, for an uneven carbon number n.

According to a preferred form, use is made of a mixture of at least two distinct volatile linear alkanes comprising from 10 to 14 carbon atoms which differ from one another by a carbon number of at least 2 and in particular of a $C_{11}/C_{13}$ mixture of volatile linear alkanes or a $C_{12}/C_{14}$ mixture of volatile linear alkanes.

Other mixtures combining more than 2 volatile linear alkanes according to the invention, such as, for example, a mixture of at least 3 distinct volatile linear alkanes comprising from 7 to 14 carbon atoms which differ from one another by a carbon number of at least 1, also come within the invention but the mixtures of 2 volatile linear alkanes according to the invention are preferred (binary mixtures), the said 2 volatile linear alkanes preferably representing more than 95% by weight and better still more than 99% by weight of the total content of volatile linear alkanes in the mixture.

According to a specific form of the invention, in a mixture of volatile linear alkanes, the volatile linear alkane having the smallest carbon number is predominant in the mixture.

According to another form of the invention, use is made of a mixture of volatile linear alkanes in which the volatile linear alkane having the greatest carbon number is predominant in the mixture.

Mention may in particular be made, as examples of mixtures suitable for the invention, of the following mixtures:

- from 50 to 90% by weight, preferably from 55 to 80% by weight, more preferably from 60 to 75% by weight, of a volatile linear $C_n$ alkane with n ranging from 7 to 14,
- from 10 to 50% by weight, preferably from 20 to 45% by weight, preferably from 24 to 40% by weight, of volatile linear $C_{n+x}$ alkane with x greater than or equal to 1, preferably x=1 or x=2, with n+x between 8 and 14,

with respect to the total weight of the alkanes in the said mixture.

In particular, the said mixture of alkanes according to the invention comprises:

- less than 2% by weight, preferably less than 1% by weight, of branched hydrocarbons,
- and/or less than 2% by weight, preferably less than 1% by weight, of aromatic hydrocarbons,
- and/or less than 2% by weight, preferably less than 1% by weight and preferentially less than 0.1% by weight of unsaturated hydrocarbons in the mixture.

More particularly, a volatile linear alkane suitable for the invention can be employed in the form of an n-undecane/n-tridecane mixture.

In particular, use will be made of a mixture of volatile linear alkanes comprising:

- from 55 to 80% by weight, preferably from 60 to 75% by weight, of volatile linear $C_{11}$ alkane (n-undecane),
- from 20 to 45% by weight, preferably from 24 to 40% by weight, of volatile linear $C_{13}$ alkane (n-tridecane),

with respect to the total weight of the alkanes in the said mixture.

According to a specific embodiment, the mixture of alkanes is an n-undecane/n-tridecane mixture. In particular, such a mixture can be obtained according to Example 1 or Example 2 of WO 2008/155059.

According to another specific embodiment, use is made of the n-dodecane sold under the reference Parafol 12-97 by Sasol.

According to another specific embodiment, use is made of the n-tetradecane sold under the reference Parafol 14-97 by Sasol.

According to yet another embodiment, use is made of a mixture of n-dodecane and n-tetradecane.

In an alternative form or additionally, the composition produced can comprise at least one volatile silicone oil or solvent compatible with a cosmetic use.

The term "silicone oil" is understood to mean an oil comprising at least one silicon atom and in particular comprising Si-O groups. According to one embodiment, the said composition comprises less than 10% by weight of nonvolatile silicone oil(s), with respect to the total weight of the composition, better still less than 5% by weight, indeed is even devoid of silicone oil.

Mention may be made, as volatile silicone oil, of cyclic polysiloxanes, linear polysiloxanes and their mixtures. Mention may be made, as volatile linear polysiloxanes, of hexamethyldisiloxane, octamethyltrisiloxane, decamethyltetrasiloxane, tetradecamethylhexasiloxane and hexadecamethylheptasiloxane. Mention may be made, as volatile cyclic polysiloxanes, of hexamethylcyclotrisiloxane, octamethylcyclotetrasiloxane, decamethylcyclopentasiloxane and dodecamethylcyclohexasiloxane.

In an alternative form or additionally, the composition produced can comprise at least one volatile fluorinated oil.

The term "fluorinated oil" is understood to mean an oil comprising at least one fluorine atom.

Mention may be made, as volatile fluorinated oil, of nonafluoromethoxybutane or perfluoromethylcyclopentane, and their mixtures.

The volatile oil can be present in a content ranging from 0.1% to 90% by weight, with respect to the total weight

of the composition, preferably ranging from 1% to 70% by weight and preferentially ranging from 5% to 50% by weight.

Nonvolatile oil

The composition according to the invention can comprise at least one nonvolatile oil.

The oil can be chosen from hydrocarbon oils, silicone oils or fluorinated oils.

Use may be made, as nonvolatile hydrocarbon oil, of liquid paraffin (or petrolatum), squalane, hydrogenated polyisobutylene (parleam oil), perhydrosqualene, mink, turtle, soybean, sweet almond, calophyllum, palm, grape seed, sesame, maize, arara, rapeseed, sunflower, cottonseed, apricot, castor, avocado, jojoba, olive or cereal germ oil; lanolic acid, oleic acid, lauric acid or stearic acid esters; fatty esters, in particular $C_{12}$-$C_{36}$, fatty esters, such as isopropyl myristate, isopropyl palmitate, butyl stearate, hexyl laurate, diisopropyl adipate, isononyl isononanoate, 2-ethylhexyl palmitate, 2-hexyldecyl laurate, 2-octyldecyl palmitate, 2-octyldodecyl myristate or lactate, di(2-ethylhexyl) succinate, diisostearyl malate, glyceryl triisostéarate or diglyceryl triisostearate; behenic acid, oleic acid, linoleic acid, linolenic acid or isostearic acid; higher fatty alcohols, in particular $C_{16}$-$C_{22}$ fatty alcohols, such as cetanol, oleyl alcohol, linoleyl alcohol, linolenyl alcohol, isostearyl alcohol or octyldodecanol; and their mixtures.

The fluorinated oils which can be used in the invention are in particular fluorosilicone oils, fluorinated polyethers or fluorosilicones, such as described in the document EP-A-847752.

The nonvolatile oil can be present in a content ranging from 0.1% to 70% by weight, with respect to the total weight of the composition, preferably ranging from 0.5% à 60% by weight and preferentially ranging from 1% to 50% by weight.

***Waxes***

The composition according to the invention can comprise, in addition to the compound or the mixture of compounds which confers, on the said composition, a dmax threading nature of greater than or equal to 5 mm, one or more additional waxes. These additional waxes alone do not contribute to the threading nature of the composition.

Use may also be made of waxes provided in the form of small particles having a size, expressed as volume-average "effective" diameter D[4,3], of the order of 0.5 to 30 micrometres, in particular of 1 to 20 micrometres and more particularly of 5 to 10 micrometres, subsequently denoted by the expression "microwaxes".

The sizes of the particles can be measured by various techniques. Mention may in particular be made of light scattering techniques (dynamic and static), Coulter counter methods, measurements by rate of sedimentation (related to the size via Stokes' law) and microscopy. These techniques make it possible to measure a particle diameter and, for some of them, a particle size distribution.

Mention may in particular be made, as microwaxes which can be used in the compositions according to the invention, of carnauba microwaxes, such as that sold under the name of MicroCare 350® by Micro Powders, synthetic wax microwaxes, such as that sold under the name of MicroEase 114S® by Micro Powders, microwaxes composed of a mixture of carnauba wax and of polyethylene wax, such as those sold under the names of MicroCare 300® and 310® by Micro Powders, microwaxes composed of a mixture of carnauba wax and of synthetic wax, such as that sold under the name MicroCare 325® by Micro Powders, polyethylene microwaxes, such as those sold under the names of Micropoly 200®, 220®, 220L® and 250S® by Micro Powders, and polytetrafluoroethylene microwaxes, such as those sold under the names of Microslip 519® and 519 L® by Micro Powders.

The composition according to the invention can comprise a content of additional waxes ranging from 0.1 to 30% by weight, with respect to the total weight of the composition; in particular, it can comprise from 0.5 to 15% by weight thereof, more particularly from 1 to 10% by weight thereof.

***Film-forming Polymer***

The composition according to the invention can comprise, in addition to the compound or the mixture of compounds which confers, on the composition, a dmax of greater than or equal to 5 mm, at least one film-forming polymer.

The film-forming polymer can be present in the composition according to the invention in a content of dry matter (or active materials) ranging from 0.1% to 30% by weight, with respect to the total weight of the composition, preferably from 0.5% to 20% by weight and better still from 1% to 15% by weight.

In the present invention, the term "film-forming polymer" is understood to mean a polymer capable of forming, alone or in the presence of an additional agent which is able to form a film, a macroscopically continuous film which adheres to keratinous fibres.

Mention may be made, among the film-forming polymers which can be used in the composition of the present invention, of synthetic polymers of radical type or of polycondensate type, polymers of natural origin, and their mixtures.

The term "radical film-forming polymer" is understood to mean a polymer obtained by polymerization of monomers possessing unsaturation, in particular ethylenic unsaturation, each monomer being capable of homopolymerizing (unlike polycondensates).

The film-forming polymers of radical type can in particular be vinyl polymers or copolymers, in particular acrylic polymers.

The film-forming vinyl polymers can result from the polymerization of monomers possessing ethylenic unsaturation having at least one acid group and/or of the esters of these acidic monomers and/or of the amides of these acidic monomers.

Use may be made, as monomer carrying an acid group, of unsaturated $\alpha,\beta$-ethylenic carboxylic acids, such as acrylic acid, methacrylic acid, crotonic acid, maleic acid or itaconic acid. Use is preferably made of (meth)acrylic acid and crotonic acid and more preferentially of (meth)acrylic acid.

The esters of acidic monomers are advantageously chosen from esters of (meth)acrylic acid (also known as (meth)acrylates), in particular alkyl (meth)acrylates, especially $C_1$-$C_{30}$ alkyl (meth)acrylates, preferably $C_1$-$C_{20}$ alkyl (meth)acrylates, aryl (meth)acrylates, in particular $C_6$-$C_{10}$ aryl (meth)acrylates, hydroxyalkyl (meth)acrylates, in particular $C_2$-$C_6$ hydroxyalkyl (meth)acrylates.

Mention may be made, among alkyl (meth)acrylates, of methyl methacrylate, ethyl methacrylate, butyl methacrylate, isobutyl methacrylate, 2-ethylhexyl methacrylate, lauryl methacrylate or cyclohexyl methacrylate.

Mention may be made, among hydroxyalkyl (meth)acrylates, of hydroxyethyl acrylate, 2-hydroxypropyl acrylate, hydroxyethyl methacrylate or 2-hydroxypropyl methacrylate.

Mention may be made, among aryl (meth)acrylates, of benzyl acrylate and phenyl acrylate.

Esters of (meth)acrylic acid which are particularly preferred are alkyl (meth)acrylates.

The alkyl group of the esters can be either fluorinated or perfluorinated, that is to say that a portion or all of the hydrogen atoms of the alkyl group are substituted by fluorine atoms.

Mention may be made, as amides of the acidic monomers, for example, of (meth)acrylamides, in particular N-alkyl(meth)acrylamides, especially N-($C_2$-$C_{12}$ alkyl)(meth)acrylamides. Mention may be made, among N-alkyl(meth)acrylamides, of N-ethylacrylamide, N-(t-butyl)acrylamide, N-(t-octyl)acrylamide and N-undecylacrylamide.

The film-forming vinyl polymers can also result from the homopolymerization or from the copolymerization of monomers chosen from vinyl esters and styrene monomers. In particular, these monomers can be polymerized with acidic monomers and/or their esters and/or their amides, such as those mentioned above.

Mention may be made, as example of vinyl esters, of vinyl acetate, vinyl neodecanoate, vinyl pivalate, vinyl benzoate and vinyl t-butylbenzoate.

Mention may be made, as styrene monomers, of styrene and $\alpha$-methylstyrene.

Mention may be made, among film-forming polycondensates, of polyurethanes, polyesters, polyesteramides, polyamides, epoxy ester resins or polyureas. The polyurethanes can be chosen from anionic, cationic, nonionic or amphoteric polyurethanes, polyurethane-acrylics, polyurethane-polyvinylpyrrolidones, polyester-polyurethanes, polyether-polyurethanes, polyureas, polyurea-polyurethanes, and their blends.

The polyesters can be obtained in a known way by polycondensation of dicarboxylic acids with polyols, in particular diols, such as described above for the sulphopolyester.

The optionally modified polymers of natural origin can be chosen from shellac resin, gum sandarac, dammars, elemis, copals, cellulose polymers and their blends.

According to a first embodiment of the composition according to the invention, the film-forming polymer can be a water-soluble polymer and can be present in an aqueous phase of the composition; the polymer is thus dissolved in the aqueous phase of the composition.

According to another alternative embodiment of the composition according to the invention, the film-forming polymer can be a polymer dissolved in a liquid fatty phase comprising oils or organic solvents, such as those described above (the film-forming polymer is then described as a fat-soluble polymer). Preferably, the liquid fatty phase comprises a volatile oil, optionally as a mixture with a nonvolatile oil, it being possible for the oils to be chosen from the oils mentioned above.

Mention may be made, as example of fat-soluble polymer, of copolymers of vinyl ester (the vinyl group being directly connected to the oxygen atom of the ester group and the vinyl ester having a saturated and linear or branched hydrocarbon radical of 1 to 19 carbon atoms bonded to the carbonyl of the ester group) and of at least one other monomer which can be a vinyl ester (other than the vinyl ester already present), an $\alpha$-olefin (having from 8 to 28 carbon atoms), an alkyl vinyl ether (the alkyl group of which comprises from 2 to 18 carbon atoms) or an allyl or methallyl ester (having a saturated and linear or branched hydrocarbon radical of 1 to 19 carbon atoms bonded to the carbonyl of the ester group).

These copolymers can be crosslinked using crosslinking agents which can be either of the vinyl type or of the allyl or methallyl type, such as tetraallyloxyethane, divinylbenzene, divinyl octanedioate, divinyl dodecanedioate and divinyl octadecanedioate.

Mention may be made, as examples of these copolymers, of the following copolymers: vinyl acetate/allyl stearate, vinyl acetate/vinyl laurate, vinyl acetate/ vinyl stearate, vinyl acetate/octadecene, vinyl acetate/octadecyl vinyl ether,

vinyl propionate/allyl laurate, vinyl propionate/vinyl laurate, vinyl stearate/1-octadecene, vinyl acetate/1-dodecene, vinyl stearate/ethyl vinyl ether, vinyl propionate/cetyl vinyl ether, vinyl stearate/allyl acetate, vinyl 2,2-dimethyloctanoate/vinyl laurate, allyl 2,2-dimethylpentanoate/vinyl laurate, vinyl dimethylpropionate/vinyl stearate, allyl dimethylpropionate/vinyl stearate, vinyl propionate/vinyl stearate, crosslinked with 0.2% of divinylbenzene, vinyl dimethylpropionate/vinyl laurate, crosslinked with 0.2% of divinylbenzene, vinyl acetate/octadecyl vinyl ether, crosslinked with 0.2% of tetraallyloxyethane, vinyl acetate/allyl stearate, crosslinked with 0.2% of divinylbenzene, vinyl acetate/1-octadecene, crosslinked with 0.2% of divinylbenzene, and allyl propionate/allyl stearate, crosslinked with 0.2% of divinylbenzene.

Mention may also be made, as fat-soluble film-forming polymers, of fat-soluble copolymers and in particular of those resulting from the copolymerization of vinyl esters having from 9 to 22 carbon atoms or of alkyl acrylates or methacrylates, the alkyl radicals having from 10 to 20 carbon atoms.

Such fat-soluble copolymers can be chosen from copolymers of poly(vinyl stearate), of poly(vinyl stearate) crosslinked using divinylbenzene, diallyl ether or diallyl phthalate, copolymers of poly(stearyl (meth)acrylate), of poly(vinyl laurate), of poly(lauryl (meth)acrylate), it being possible for these poly(meth)acrylates to be crosslinked using ethylene glycol dimethacrylate or tetraethylene glycol dimethacrylate.

The fat-soluble copolymers defined above are known and are described in particular in Application FR-A-2 232 303; they can have a weight-average molecular weight ranging from 2000 to 500 000 and preferably from 4000 to 200 000.

Mention may also be made, as fat-soluble film-forming polymers which can be used in the invention, of polyalkylenes and in particular copolymers of C2-C20 alkenes, such as polybutene, alkylcelluloses with a saturated or unsaturated and linear or branched C1 to C8 alkyl radical, such as ethylcellulose and propylcellulose, copolymers of vinylpyrrolidone (VP) and in particular copolymers of vinylpyrrolidone and of C2 to C40 alkene and better still C3 to C20 alkene. Mention may be made, as example ofVP copolymer which can be used in the invention, of the VP/vinyl acetate, VP/ethyl methacrylate, VP/ethyl methacrylate/methacrylic acid, VP/eicosene, VP/hexadecene, VP/triacontene, VP/styrene or VP/acrylic acid/lauryl methacrylate copolymer or butylated polyvinylpyrrolidone (PVP).

Mention may also be made of silicone resins, generally soluble or swellable in silicone oils, which are crosslinked polyorganosiloxane polymers. The nomenclature of silicone resins is known under the name of "MDTQ", the resin being described according to the various siloxane monomer units which it comprises, each of the letters "MDTQ" characterizing one type of unit.

Mention may be made, as examples of commercially available polymethylsilsesquioxane resins, of those which are sold:

by Wacker under the reference Resin MK, such as Belsil PMS MK;

by Shin-Etsu under the reference KR-220L.

Mention may be made, as siloxysilicate resins, of trimethylsiloxysilicate (TMS) resins, such as those sold under the reference SR1000 by General Electric or under the reference TMS 803 by Wacker. Mention may also be made of trimethylsiloxysilicate resins sold in a solvent, such as cyclomethicone, sold under the name "KF-7312J" by Shin-Etsu or "DC 749" or "DC 593" by Dow Coming.

Mention may also be made of copolymers of silicone resins, such as those mentioned above, with polydimethylsiloxanes, such as the pressure-sensitive adhesive copolymers sold by Dow Coming under the reference BIO-PSA and described in the document US 5 162 410, or also the silicone copolymers resulting from the reaction of a silicone resin, such as those described above, and of a diorganosiloxane, such as are described in the document WO 2004/073626.

The film-forming polymer can also be present in the composition in the form of particles in dispersion in an aqueous phase or in a nonaqueous solvent phase, generally known under the name of latex or pseudolatex. The techniques for the preparation of these dispersions are well known to a person skilled in the art.

Use may be made, as aqueous film-forming polymer dispersion, of acrylic dispersions, sold under the names Neocryl XK-90®, Neocryl A-1070®, Neocryl A-1090®, Neocryl BT-62®, Neocryl A-1079® and Neocryl A-523® by Avecia-Neoresins, Dow Latex 432® by Dow Chemical, Daitosol 5000 AD® or Daitosol 5000 SJ® by Daito Kasey Kogyo; Syntran 5760® by Interpolymer, Allianz OPT by Röhm & Haas, aqueous dispersions of acrylic or styrene/acrylic polymers, sold under the trade name Joncryl® by Johnson Polymer, or also aqueous dispersions of polyurethane, sold under the names Neorez R-981® and Neorez R-974® by Avecia-Neoresins, Avalure UR-405®, Avalure UR-410®, Avalure UR-425®, Avalure UR-450®, Sancure 875®, Sancure 861®, Sancure 878® and Sancure 2060® by Goodrich, Impranil 85® by Bayer, Aquamere H-1511® by Hydromer; sulphopolyesters, sold under the trade name Eastman AQ® by Eastman Chemical Products, vinyl dispersions, such as Mexomer PAM® from Chimex, and their blends.

Mention may be made, as examples of nonaqueous dispersions of film-forming polymer, of acrylic dispersions in isododecane, such as Mexomer PAP® from Chimex, dispersions of particles of a grafted ethylenic polymer, preferably an acrylic polymer, in a liquid fatty phase, the ethylenic polymer advantageously being dispersed in the absence of additional stabilizer at the surface of the particles, such as described in particular in the document WO 04/055081.

The composition according to the invention can comprise a plasticizing agent which promotes the formation of a film with the film-forming polymer. Such a plasticizing agent can be chosen from all the compounds known to a person

skilled in the art as being capable of performing the desired function.

The composition can also comprise ingredients commonly used in cosmetics, such as lipophilic gelling agents, colouring materials, fillers, fibres and their mixtures.

***Colouring material***

The composition according to the invention can also comprise at least one colouring material, such as pulverulent materials, fat-soluble dyes or water-soluble dyes.

The pulverulent colouring materials can be chosen from pigments and pearlescent agents.

The pigments can be white or coloured, inorganic and/or organic and coated or noncoated. Mention may be made, among inorganic pigments, of titanium dioxide, optionally treated at the surface, zirconium, zinc or cerium oxides, and also iron or chromium oxides, manganese violet, ultramarine blue, chromium hydrate and ferric blue. Mention may be made, among organic pigments, of carbon black, pigments of D & C type and lakes, based on cochineal carmine, of barium, strontium, calcium or aluminium.

The pearlescent agents can be chosen from white pearlescent pigments, such as mica covered with titanium oxide or with bismuth oxychloride, coloured pearlescent pigments, such as titanium oxide-coated mica with iron oxides, titanium oxide-coated mica with in particular ferric blue or chromium oxide or titanium oxide-coated mica with an organic pigment of the abovementioned type, and pearlescent pigments based on bismuth oxychloride.

The fat-soluble dyes are, for example, Sudan red, D&C Red 17, D&C Green 6, β-carotene, soybean oil, Sudan brown, D&C Yellow 11, D&C Violet 2, D&C Orange 5, quinoline yellow or annatto.

These colouring materials can be present in a content ranging from 0.01 to 30% by weight, with respect to the total weight of the composition.

***Fillers***

The composition according to the invention can additionally comprise at least one filler.

The fillers can be chosen from those well known to a person skilled in the art and commonly used in cosmetic compositions. The fillers can be inorganic or organic and lamellar or spherical. Mention may be made of talc, mica, silica, kaolin, powders formed of polyamide, such as Nylon®, sold under the name Orgasol® by Atochem, of poly-β-alanine and of polyethylene, powders formed of tetrafluoroethylene polymers, such as Teflon®, lauroyl lysine, starch, boron nitride, hollow polymer microsphères which are expanded, such as those of poly(vinylidene chloride/acrylonitrile), for example those sold under the name Expancel® by Nobel Industrie, acrylic powders, such as those sold under the name Polytrap® by Dow Coming, particles formed of poly(methyl methacrylate) and silicone resin microbeads (Tospearls® from Toshiba, for example), precipitated calcium carbonate, magnesium carbonate, basic magnesium carbonate, hydroxyapatite, hollow silica microspheres (Silica Beads® from Maprecos), glass or ceramic microcapsules, or metal soaps derived from organic carboxylic acids having from 8 to 22 carbon atoms and in particular from 12 to 18 carbon atoms, for example zinc stearate, magnexium stearate, lithium stearate, zinc laurate or magnesium myristate.

Use may also be made of a compound capable of swelling when heated and in particular of heat-expandable particles, such as nonexpanded microspheres formed of vinylidene chloride/acrylonitrile/methyl methacrylate copolymer or of copolymer of homopolymer of acrylonitrile, such as, for example, those sold respectively under the references Expancel® 820 DU 40 and Expancel®007WU by Akzo Nobel.

The fillers can represent from 0.1 to 25% by weight, in particular from 1 to 20% by weight, with respect to the total weight of the composition. The composition of the invention can additionally comprise any additive conventionally used in cosmetics, such as antioxidants, preservatives, fibres, fragrances, neutralizing agents, gelling agents, thickeners, vitamins, coalescence agents, plasticizers and their mixtures.

***Fibres***

The composition according to the invention can comprise, in addition to the compound or the mixture of compounds which confers, on the said composition, a dmax threading nature of greater than or equal to 5 mm, "additional" fibres. These additional fibres alone do not contribute to the threading nature of the composition.

The additional fibres can be present in the composition according to the invention in a content ranging from 0.01% to 10% by weight, with respect to the total weight of the composition, in particular from 0.1% to 5% by weight and more particularly from 0.3% to 3% by weight.

***Cosmetic active principles***

Mention may in particular be made, as cosmetic active principles which can be used in the compositions

according to the invention, of antioxidants, preservatives, fragrances, neutralizing agents, emollients, moisturizing agents, vitamins and screening agents, in particular sunscreens.

Of course, a person skilled in the art will take care to choose the optional additional additives and/or their amounts so that the advantageous properties of the composition according to the invention are not, or not substantially, detrimentally affected by the envisaged addition.

## MAKE-UP-REMOVING AND/OR CLEANSING COMPOSITION

This make-up-removing and/or cleansing composition can be water or a water-soluble solvent, such as, for example, the product Effacil® from Lancôme. The make-up-removing and/or cleansing composition can comprise essentially water and/or one or more water-soluble solvent(s), for example present at an amount of greater than or equal to 95% by weight, with respect to the total weight of the composition, and in particular ranging from 80 to 100% by weight, with respect to the total weight of the composition.

In an alternative form, this composition can comprise an oily phase, such as the product Bi-facil® from Lancôme. The make-up-removing and/or cleansing composition can comprise an oily phase present in an amount of greater than or equal to 95% by weight, with respect to the total weight of the composition, and in particular ranging from 80 to 100% by weight, with respect to the total weight of the composition. Such a composition can optionally consist of a mixture of an oily phase with an aqueous phase comprising water and/or one or more water-soluble solvent(s).

In order to carry out this make-up removal, at least one finger must be impregnated with this composition. In an alternative form, a substrate can be impregnated and can thus be dry or anhydrous. In an alternative form, this substrate can be preimpregnated with the composition.

This substrate can be a woven or a nonwoven material which is optionally laminated, such as a Demak'up® disc or a square wipe of Wypall L40® type from Kimberly-Clark.

## MAKE-UP-REMOVING AND/OR CLEANSING KIT

This kit can comprise a make-up composition according to the invention in combination with a make-up-removing and/or cleansing composition.

This make-up-removing and/or cleansing composition can be packaged with the said make-up composition, separately, in one and the same packaging item.

The make-up-removing and/or cleansing composition can be as described above.

## COMBINATION

The composition according to the invention can be packaged in a packaging and applicational combination comprising:

i) an applicator,
ii) a packaging device intended to house the said composition, and
iii) a heating device.

This packaging and applicational combination can optionally be provided in the form of a kit, it being possible for the applicator, the packaging device and/or the heating device to be housed separately in one and the same packaging item.

### Applicator

The applicator or applicational device can comprise a grasping component connected to a support, optionally detachable, suitable for receiving the said composition. This support can comprise applicational means and in particular combing means. This grasping component or this support can house a heating device suitable for heating the said composition.

The applicational means can comprise means which make it possible to smooth and/or separate the eyelashes or eyebrows, in particular in the form of teeth, bristles or other embossments.

The applicator is arranged in order to apply the composition to the eyelashes or the eyebrows and can comprise, for example, a brush or a comb.

The applicator can also be used for the finishing of the make-up, over a region of the eyelashes or eyebrows made up or laden with the composition.

The brush can comprise a twisted core and bristles taken between the turns of the core or can be made in

another way again.

The comb is, for example, produced from a single part by moulding of plastic.

In some embodiments, the applicational element is fitted to the end of a rod which can be flexible, which can contribute to improving the comfort on application.

## Packaging device

The packaging device comprises a container housing the make-up and/or care composition. This composition can then be withdrawn from the container by immersing an applicator in the latter.

This applicator can be integral with a closure element of the container. This closure element can form a component for grasping the applicator. This grasping component can form a cap to be fitted in detachable fashion to the said container by any appropriate means, such as by screwing, snapping, coupling or other. Such a container can thus reversibly house the said applicator.

This container can optionally be equipped with a wringer suitable for removing a surplus of product withdrawn by the applicator.

## Heating device

The composition according to the invention can be heated by means of a heating device integral with the applicator or with the packaging device or external to the said combination.

The heating device employed can be an artificial source of heat, that is to say a source of heat other than human or animal heat, the heat of the sun or the heat which can result from the rubbing of the applicational surface over the keratinous fibres during application. The source of heat can be other than the heat produced by combustion in ambient air of a fuel, for example the flame of a lighter or of a candle.

The introduction of heat in order to heat up the composition results, for example, in a local rise of at least 5°C in its temperature, better still of at least 10°C, indeed even of 20°C or 30°C.

According to a specific embodiment, the composition is solid and is heated prior to the application thereof, it being possible for the heating device used to be the applicator itself. Thus, in the case of a mascara, the composition can be applied using a heating brush.

According to another embodiment, the composition is heated during the application thereof. In such a case, the heating device used is generally the applicator itself. Thus, in the case of a mascara, the composition can be applied using a heating brush or comb. In such a case, the heating device associated with the applicator can be arranged so as not to substantially heat the grasping component of the applicator.

According to yet another embodiment, the composition is heated subsequent to the application thereof. According to a first alternative form, the composition can be heated using means not specifically intended for a heating operation, such as, for example, a body which is occasionally warm. According to a second alternative form of this embodiment, the composition can be heated using a device specifically devoted to the heating. It can in particular be a means which propels hot air, such as a hairdrier, or a heating device, for example as described below.

According to yet another embodiment, the heating device is formed by a device separate from the applicator, the combination being configured in the form of a packaging and applicational device additionally comprising a container comprising a composition in accordance with the invention. Such a device can be packaged inside a packaging of the blister pack type. The heating means can be of the type of those described in Patents US 6 009 884 or US 5 853 010. Other devices configured in the form of heating tongs (in the case of the eyelashes) can also be used. Such devices are described in particular in Patent US 6 220 252.

The piece of product can be heated over an external surface and can thus retain, at the core, a temperature of less than or equal to 30°C during application for an ambient temperature of 20°C, at least when the piece of product comprises the starting amount of product.

The temperature M.p. to which the external surface of the product is heated can be greater than or equal to 40°C, for example 50°C, for example 60°C, for example 65°C. The temperature M.p. can advantageously be less than or equal to 60°C. It can in particular be between 40°C and 60°C.

The introduction of energy in order to soften the composition can take place for less than 60 s, better still less than 30 s, indeed even less than 10 s. The heating of the product can take place without the product flowing through the openings of the device.

The product can be heated up by being exposed to infrared radiation or to radio frequency radiation, be heated up by blowing hot air, be heated up by being exposed to ultrasonic vibrations or be heated up by transfer of heat on contact with or close to a hot surface. The latter can rest axially and/or radially against the applicational surface, for example the end of the stick when the piece of product is in the stick form. The heating device can heat up the product in a controlled manner, by virtue of temperature regulation.

The application of the make-up composition can take place without the surface of the softened product passing through a wringing component.

Examples appearing below are presented by way of illustration and without implied limitation of the invention. Unless otherwise indicated, the amounts shown are expressed as percentage by weight.

## IMPLEMENTATIONAL EXAMPLES

Several compositions were formulated and tested according to a specific protocol for the purpose of demonstrating the advantage conferred by the presence of at least one silicone oil in accordance with the invention in make-up compositions according to the invention from a point of make-up removal of the said composition.

### Protocol for making up and removing make-up and evaluation of the make-up removal

In order to evaluate the make-up-removing and/or cleansing result for the composition according to the present patent application, an in vitro test is carried out according to the following protocol:

- The make-up composition according to the invention is heated to a temperature, for example, of between 65 and 75°C using an applicator equipped with an appropriate heating device.
- Once the temperature has been reached, the composition is applied to 3 test specimens of straight 30-knot Caucasian hair (60 eyelashes 1 cm long), fringe length of 2 cm, by carrying out 3 × 3 passes 2 minutes apart with product being taken up between each series of 3 in order to obtain complete coating of the hair by the said composition, resulting in a deposition of an amount of from 20 to 30 mg of make-up composition per test specimen.
- Each test specimen is subsequently dried at ambient temperature for a drying time of one hour.
- Make-up is then removed from the 3 made-up test specimens with a Bifacil® lotion, an amount of 1.5 ml of the said lotion being deposited on a Demak'up® cotton disc.
- In order to do this, the 3 test specimens are then deposited on the said respective cotton substrate of the Demak'up® disc type impregnated with the said lotion and then each cotton disc is folded in 2 around its respective test specimen.
- A gentle pressure is applied to each cotton disc and the said cotton discs are pulled away relative to the test specimens.
- The deposition on each cotton disc is then observed visually.
- This make-up-removing operation is repeated as many times as necessary until complete removal of make-up from the test specimens is obtained or, if appropriate, until the test specimens break.

A test specimen is stated as being able to be freed from make-up when complete removal of make-up is observed with the use of at most 15 make-up-removing cotton discs to remove the make-up composition from the test specimens and when the latter emerges intact therefrom. A result is considered satisfactory when at most 10 cotton discs are used to completely remove the make-up composition from the test specimen. A result is considered highly satisfactory when at most 5 cotton discs are used to completely remove the make-up composition from the test specimen.

A test specimen is stated as being unable to be freed from make-up when complete removal of make-up could not be after use of 15 make-up-removing cotton discs to remove the make-up composition from the test specimens or else when the test specimens emerge broken during a make-up-removing stage.

Preparation of the formulations tested according to the first aspect of the invention:

The formulations prepared were tested according to the protocol for making up and removing make-up described above.

Examples outside the invention

| Compounds | Formulation 1 outside the invention (%) | Formulation 2 outside the invention (%) | Formulation 3 outside the invention (%) | Formulation 4 outside the invention (%) | Supplier | Commercial name |
|---|---|---|---|---|---|---|
| Black iron oxide | 3 | 4 | 4 | 4 | Sun | Sunpuro Black Iron Oxide® |
| Ethylene/VA copolymer | 97 | 81 | 81 | 81 | National Starch | Cool Bind 34-1300® |
| Polydimethylsiloxane comprising $\alpha,\omega$-($C_{30}$-$C_{45}$ alkyl) groups | ----- | 15 | ----- | ----- | Momentive Performance Materials | SF 1642® |
| Oxyethylenated (20 EO)/oxypropylenated (20 PO) polydimethylsiloxane (DP : 170 -, Viscosity : 1000 cSt) | ----- | ----- | 15 | ----- | Dow Corning | Dow Corning Q 2-5220 Resin Modifier ® |
| Mixture of polyglycerolated vegetable waxes (mimosa/ jojoba/sunflower) | ----- | ----- | ----- | 15 | Gattefossé | Hydracire S® |
| | Unable to be freed from make-up | Unable to be freed from make-up | Unable to be freed from make-up | Unable to be freed from make-up | | |

| Compounds | Formulation 5 outside the invention (%) | Formulation 6 according to the invention (%) | Formulation 7 according to the invention (%) | Supplier | Commercial name |
|---|---|---|---|---|---|
| Black iron oxide | 4 | 4 | 4 | Sun | Sunpuro Black Iron Oxide® |
| Ethylene/VA copolymer | 81 | 81 | 81 | National Starch | Cool Bind 34-1300® |
| Polydimethylsiloxane 350 cSt | 15 | ----- | ----- | Wacker | Belsil 350 PDM® |
| Polydimethylsiloxane 100 cSt | ----- | 15 | ----- | Dow Corning | 200R Fluid 100 cSt® |
| Polydimethylsiloxane 5 cSt | ----- | ----- | 15 | Dow Corning | 200R Fluid 5 cSt® |
| | Unable to be freed from make-up | Able to be freed from make-up<br>Highly satisfactory | Able to be freed from make-up<br>Highly satisfactory | | |

Results :

The use of the noncyclic silicone oils with a viscosity at 25°C of less than or equal to 300 cSt in the formulations 6 and 7 provides easy and significant make-up removal of this make-up composition and in particular of the compound which confers, on the said composition, a dmax threading nature of greater than or equal to 5 mm, in this case the ethylene/vinyl acetate copolymer, using the Bifacil® cotton pad.

By contrast, the other compounds of the formulations outside the invention 1 to 5 do not make it possible to observe any removal of make-up from the made-up test specimens using the Bifacil® cotton pad or else the make-up

removal observed does not prove to be significant.

Preparation of the formulations tested according to the second aspect of the invention:

The formulations prepared were tested according to the protocol for making up and removing make-up described above.

Examples outside the invention

| Compounds | Formulation 1 outside the invention (%) | Formulation 2 outside the invention (%) | Formulation 3 outside the invention (%) | Formulation 4 outside the invention (%) | Supplier | Commercial name |
|---|---|---|---|---|---|---|
| Black iron oxide | 3 | 4 | 4 | 4 | Sun | Sunpuro Black Iron Oxide® |
| Ethylene/VA copolymer | 97 | 81 | 81 | 81 | National Starch | Cool Bind 34-1300® |
| Polydimethylsiloxane comprising α,ω-($C_{30}$-$C_{45}$ alkyl) groups | ----- | 15 | ----- | ----- | Momentive Performance Materials | SF 1642® |
| Oxyethylenated (20 EO)/oxypropylenated (20 PO) polydimethylsiloxane (DP : 170 –, Viscosity : 1000 cSt) | ----- | ----- | 15 | ----- | Dow Corning | Dow Corning Q 2-5220 Resin Modifier ® |
| Mixture of polyglycerolated vegetable waxes (mimosa/ jojoba/sunflower) | ----- | ----- | ----- | 15 | Gattefossé | Hydracire S® |
| | Unable to be freed from make-up | Unable to be freed from make-up | Unable to be freed from make-up | Unable to be freed from make-up | | |

Examples according to the invention

| Compounds | Formulation 5 according to the invention (%) | Formulation 6 according to the invention (%) | Supplier | Commercial name |
| --- | --- | --- | --- | --- |
| Black iron oxide | 4 | 4 | Sun | Sunpuro Black Iron Oxide® |
| Ethylene/VA copolymer | 81 | 81 | National Starch | Cool Bind 34-1300® |
| polydimethylsiloxane carrying triglycerol groups and $C_{12}$ alkyl groups | 15 | ----- | Shin-Etsu | KF-6105® |
| Polyglyceryl-3 polydimethylsiloxyethyl dimethicone | ----- | 15 | Shin-Etsu | KF-6104® |
| | Able to be freed from make-up<br>Highly satisfactory | Able to be freed from make-up<br>Highly satisfactory | | |

Results:

The use of the silicone oils carrying at least one glycerol group in the make-up composition according to the invention, as employed in the compositions 5 and 6, provide easy and significant make-up removal of this make-up composition and in particular of the compound which confers, on the said composition, a dmax threading nature of greater than or equal to 5 mm, in this case the ethylene/vinyl acetate copolymer, using the Bifacil® cotton pad.

By contrast, the other compounds of the compositions outside the invention 1 to 4 do not make it possible to observe any removal of make-up from the made-up test specimens using the Bifacil® cotton pad or else the make-up removal observed does not prove to be significant.

Preparation of the formulations tested according to the third aspect of the invention:

The formulations prepared were tested according to the protocol for making up and removing make-up described above.

| Compounds | Formulation 1 outside the invention (%) | Formulation 2 outside the invention (%) | Formulation 3 outside the invention (%) | Formulation 4 outside the invention (%) | Supplier | Commercial name |
|---|---|---|---|---|---|---|
| Black iron oxide | 3 | 4 | 4 | 4 | Sun | Sunpuro Black Iron Oxide® |
| Ethylene/VA copolymer | 97 | 81 | 81 | 81 | National Starch | Cool Bind 34-1300® |
| Polydimethylsiloxane comprising α,ω-($C_{30}$-$C_{45}$ alkyl) groups | ----- | 15 | ----- | ----- | Momentive Performance Materials | SF 1642® |
| Oxyethylenated (20 EO)/oxypropylenated (20 PO) polydimethylsiloxane (DP : 170 –, Viscosity : 1000 cSt) | ----- | ----- | 15 | ----- | Dow Corning | Dow Corning Q 2-5220 Resin Modifier ® |
| Mixture of polyglycerolated vegetable waxes (mimosa/ jojoba/sunflower) | ----- | ----- | ----- | 15 | Gattefossé | Hydracire S® |
| | Unable to be freed from make-up | Unable to be freed from make-up | Unable to be freed from make-up | Unable to be freed from make-up | | |

| Compounds | Formulation 5 outside the invention (%) | Formulation 6 outside the invention (%) | Formulation 7 according to the invention (%) | Supplier | Commercial name |
|---|---|---|---|---|---|
| Black iron oxide | 4 | 4 | 4 | Sun | Sunpuro Black Iron Oxide® |
| Ethylene/VA copolymer | 81 | 81 | 81 | National Starch | Cool Bind 34-1300® |
| Phenyltrimethylsiloxy-trisiloxane | 15 | ----- | ----- | Dow Corning | DC556® |
| Trimethylpentaphenyl-trisiloxane | ----- | 15 | ----- | Wacker | Belsil 200 PDM® |
| Trimethylsiloxyphenyl dimethicone | ----- | ----- | 15 | Wacker | Belsil 1000 PDM® |
| | Unable to be freed from make-up | Unable to be freed from make-up | Able to be freed from make-up Highly satisfactory | | |

Results:

The use of at least one phenylated silicone oil of high viscosity in the make-up composition according to the invention, as employed in the composition 7, provides easy and significant make-up removal of this composition and in particular of the compound which confers, on the said composition, a dmax threading nature of greater than or equal to 5 mm, in this case the ethylene/vinyl acetate copolymer, using the Bifacil® cotton pad.

By contrast, the other compounds of the compositions 1 to 6 outside the invention do not make it possible to observe any removal of make-up from the made-up test specimens using the Bifacil® cotton pad or else the make-up removal observed does not prove to be significant.

Throughout the patent application, the wording "comprising a" means "comprising at least one", unless otherwise specified.

**Claims**

1. Composition for making up keratinous fibres, comprising:

- at least one compound or one mixture of compounds capable of conferring, on the said composition, a dmax threading nature of greater than or equal to 5 mm, and
- at least one silicone polymer of following general formula (I'):

$$R^1_a\,R^2_b\,R^3_c\,SiO_{(4-a-b-c)/2} \qquad (I')$$

in which formula (I'):

○ a varies from 1 to 2.5, and b and c, independently of one another, vary from 0.001 to 1.5;
○ $R^1$, identical or different, is chosen from:

- i) $C_1$ to $C_{30}$ alkyl radicals, which is optionally substituted by one or more fluorine atoms, amino groups and/or carboxyl groups,
- ii) aryl or aralkyl radicals,
- iii) radicals of general formula (II'):

$$-C_dH_{2d}-O-(C_2H_4O)_e(C_3H_6O)_fR^4 \qquad (II')$$

in which formula (II'):

- $R^4$ being a $C_1$ to $C_{30}$ hydrocarbon radical or an $R^5$-(CO)- radical with $R^5$ being a $C_1$ to $C_{30}$ hydrocarbon radical, and
- d, e and f being integers such that d varies from 0 to 15, and e and f, independently of one another, vary from 0 to 50, and
- iv) their combinations,

○ $R^2$ is a radical represented by the general formula (III'):

$$-Q-O-X \qquad (III')$$

in which formula (III'):

- Q being a divalent $C_2$ to $C_{20}$ hydrocarbon radical which can include at least one ether bond and/or at least one ester bond, and
- X being a polyhydroxylated hydrocarbon radical, in particular a polyhydroxy($C_1$-$C_6$)alkyl radical, such as $-[CH_2-CH(OH)-CH_2-O]_p-H$ with p between 1 and 10, preferably equal to 3,

○ $R^3$ is an organosiloxane group of general formula (IV'):

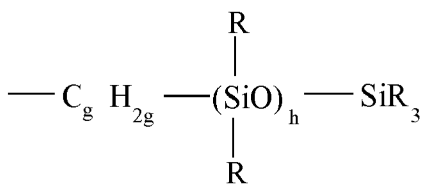

(IV')

in which formula (IV'):
- each of the R radicals representing, independently of one another, a radical chosen from $C_1$ to $C_{30}$ alkyl radicals, which is optionally substituted by one or more fluorine atoms, and aryl and aralkyl radicals,
- g and h being integers such that g varies from 1 to 5 and h varies from 0 to 500.

2. Composition for making up keratinous fibres according to Claim 1, in which the composition is capable of being brought to a temperature of greater than or equal to 45°C, better still of greater than or equal to 55°C and even

better still of greater than or equal to 65°C.

**3.** Composition for making up keratinous fibres according to Claim 1 or 2, in which the compound or the mixture of compounds conferring, on the said composition, a dmax threading nature of greater than or equal to 5 mm is chosen from:

a) polymers and copolymers comprising at least one alkene monomer, in particular ethylene-based copolymers,
b) poly(vinyl acetate) homopolymers,
c) silicone resins,
d) film-forming block ethylenic polymers, which preferably comprise at least one first block and at least one second block having different glass transition temperatures (Tg), the said first and second blocks being connected to one another via an intermediate block comprising at least one constituent monomer of the first block and at least one constituent monomer of the second block,
e) copolymers of dienes and of styrene,
f) sulphopolyesters,
g) waxes,
h) fibres,
and their mixtures.

**4.** Composition for making up keratinous fibres according to Claim 1, 2 or 3, in which the composition is in the solid form and exhibits a hardness in shearing at 20°C of greater than or equal to 375 g/m and in particular of between 375 g/m and 15 000 g/m.

**5.** Composition for making up keratinous fibres according to any one of the preceding claims, **characterized in that** the said composition is a mascara.

**6.** Composition for making up keratinous fibres according to any one of the preceding claims, in which the compound or the mixture of compounds conferring, on the said composition, a dmax threading nature of greater than or equal to 5 mm is chosen from:

- copolymers of alkene and of vinyl acetate, in particular copolymers of ethylene and of vinyl acetate,
- copolymers of ethylene and of octene,
- poly(vinyl acetate) homopolymers,
- T silicone resins, such as polyphenylsiloxanes,
- film-forming block ethylenic copolymers resulting essentially from monomers chosen from alkyl methacrylates, alkyl acrylates and their mixtures,
- copolymers of butadiène and of styrene,
- copolymers obtained by condensation of diethylene glycol, cyclohexanedimethanol, isophthalic acid and sulphoisophthalic acid,

and their blends.

**7.** Composition for making up keratinous fibres according to any one of the preceding claims, in which the compound or the mixture of compounds conferring, on the said composition, a dmax threading nature of greater than or equal to 5 mm is chosen from polymers and copolymers comprising at least one alkene monomer and poly(vinyl acetate) homopolymers.

**8.** Composition for making up keratinous fibres according to any one of the preceding claims, in which the compound or the mixture of compounds conferring, on the said composition, a dmax threading nature of greater than or equal to 5 mm is present in a content of dry matter ranging from 5 to 100% by weight, with respect to the total weight of the composition, preferably from 10 to 100% by weight, better still from 25 to 100% by weight, indeed even from 50 to 100% by weight, or also from 80 to 100% by weight, with respect to the total weight of the composition.

**9.** Composition for making up keratinous fibres according to any one of the preceding claims, in which the silicone polymer of general formula (I') is such that:

○ a varies from 1 to 1.4, and b and c, independently of one another, vary from 0.02 to 0.03, and
○ $R^1$ is a $C_1$ to $C_{10}$ alkyl radical,
○ $R^2$ is represented by the formula (IIIA'):

$$C_3H_6O[CH_2CH(OH)CH_2O]_n\,H \qquad \text{(IIIA')}$$

in which formula (IIIA'):

- n varies from 1 to 5, and

○ $R^3$ is represented by the formula (IVA'):

$$-C_2H_4(CH_3)_2SiO[(CH_3)_2SiO]_m\,Si(CH_3)_3 \qquad \text{(IVA')}$$

in which formula (IVA'):

- m varies from 3 to 9.

**10.** Composition for making up keratinous fibres according to any one of the preceding claims, in which the silicone polymer of general formula (I') is chosen from:

- i) polyglycerol-3 polydimethylsiloxyethyl dimethicone of formula (V'):

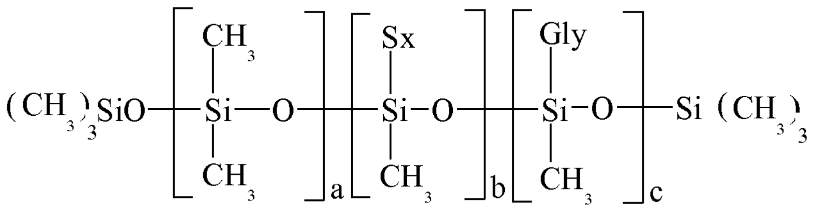

(V')

in which formula (V'):

Sx: $-C_2H_4\,[(CH_3)_2SiO]_mSi(CH_3)_3$
Gly: $-C_3H_6O[CH_2\text{-}CH(OH)CH_2O]_nH$
with a = 1-1.4, b = 0.02-0.04, c = 0.02-0.04, m = 3-9 and n = 1-5;

- ii) lauryl polyglyceryl-3 polydimethylsiloxyethyl dimethicone of formula (VI'):

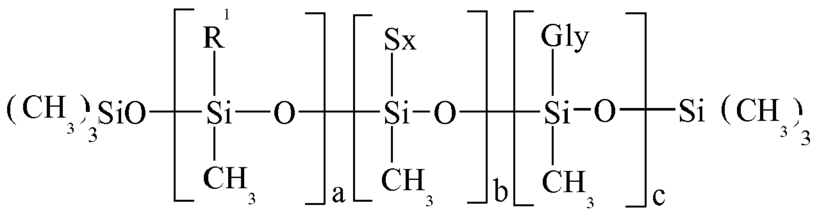

(VI')

in which formula (VI'):

Sx: $-C_2H_4\,[(CH_3)_2SiO]_mSi(CH_3)_3$
Gly: $-C_3H_6O[CH_2\text{-}CH(OH)CH_2O]_nH$
with a = 1-1.4, b = 0.02-0.04, c = 0.02-0.04, m = 3-9 and n = 1-5, and
$R^1$ is a methyl radical or a lauryl radical; and

- iii) polyglyceryl-3 disiloxane dimethicone of formula (VII'):

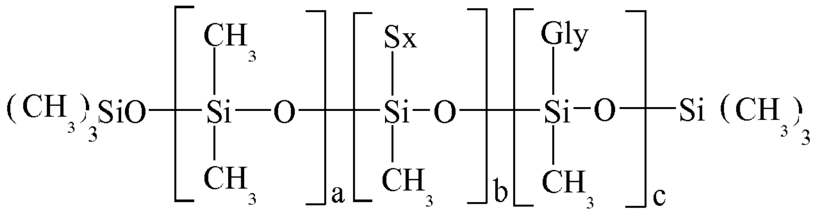 (VII')

in which formula (VII'):

Sx: $-O(CH_3)_2SiO-Si(CH_3)_3$
Gly: $-C_3H_6O[CH_2-CH(OH)CH_2O]_nH$
with a = 1-1.4, b = 0.02-0.04, c = 0.02-0.04 and n = 1-5.

**11.** Composition for making up keratinous fibres according to any one of the preceding claims, in which the silicone polymer is of following formula (VIII'):

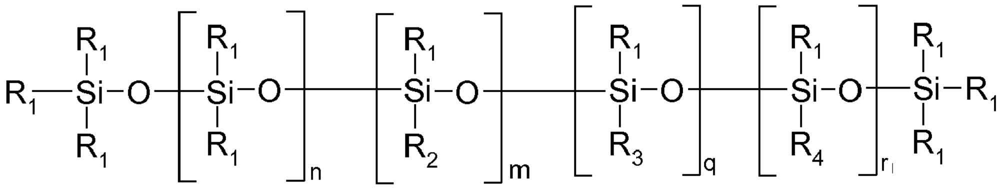

(VIII')

in which formula (VIII'):

○ $R_1$, which are identical or different, represent:

- i) a linear or branched $C_1$-$C_{20}$ alkyl group; or
- ii) a hydroxyl group;

○ $R_2$ represents the polysiloxane group $-ALK-[Si(R_1)_2-O]_{n'}-Si(R_1)_3$, with $R_1$ as defined above and ALK representing a divalent $C_1$-$C_6$ alkylene group;
○ $R_3$ represents the $-ALK'-O-[CH_2-CH(OH)-CH_2-O]_p-H$ group, with p between 1 and 10; and ALK' representing a divalent $C_1$-$C_6$ alkylene group;
○ $R_4$ represents a $C_1$-$C_{20}$ alkyl group which is linear or branched;
○ m and r, which are identical or different, are an integer inclusively between 0 and 150;
○ n, n' and q, which are identical or different, are an integer inclusively between 1 and 300.

**12.** Composition for making up keratinous fibres according to any one of the preceding claims, in which the silicone polymer is chosen from following formula (IX'):

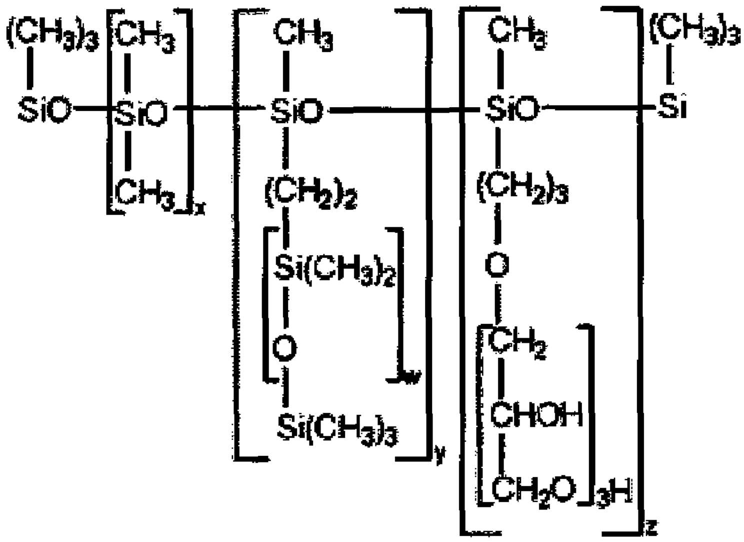

in which formula (IX'):

x, y and z, which are identical or different, are an integer inclusively between 1 and 25, and w is an integer between 1 and 25.

or from following formula (X'):

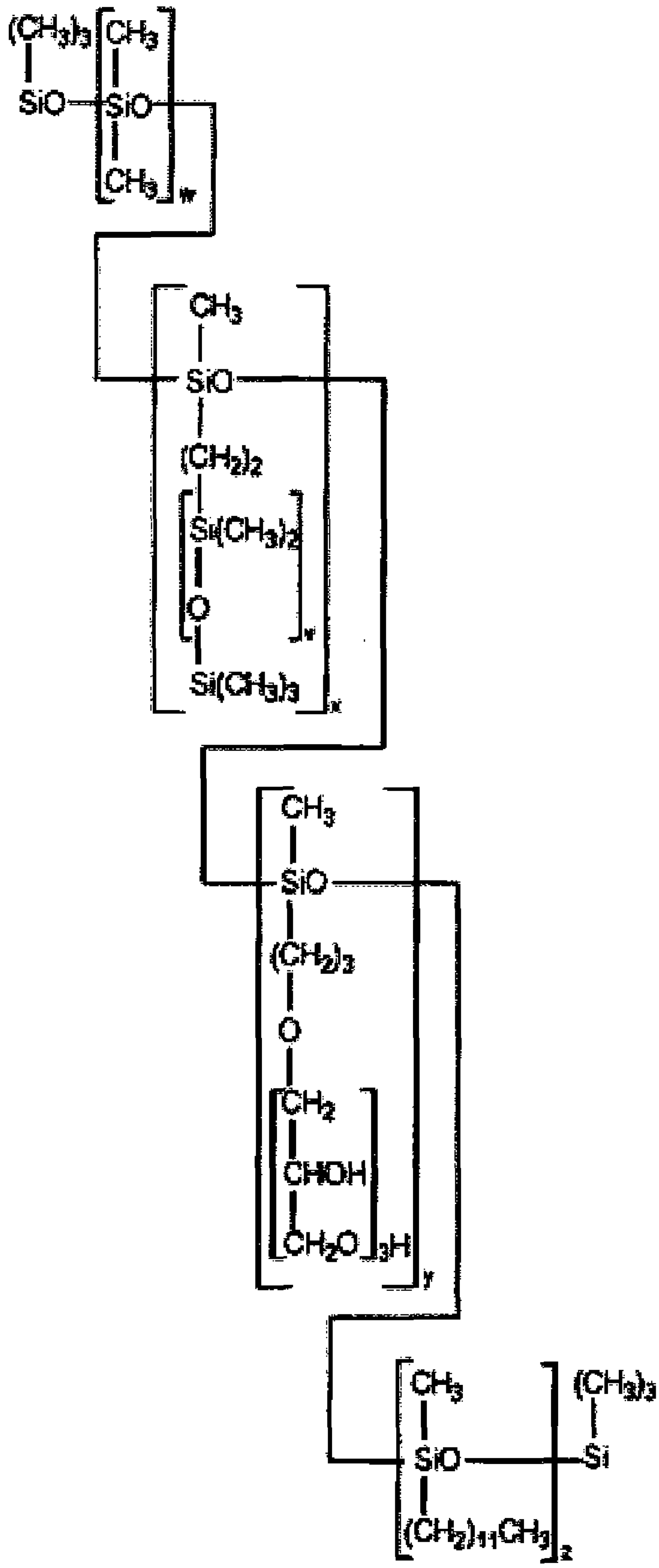

in which formula (X'):

x, y and z, which are identical or different, are an integer inclusively between 1 and 25,

and w is an integer between 1 and 25.

**13.** Composition for making up keratinous fibres according to any one of the preceding claims, in which the silicone polymer is present in a content ranging from 0.1 to 40% by weight, with respect to the total weight of the composition.

**14.** Composition for making up keratinous fibres according to any one of the preceding claims, **characterized in that** the said composition includes less than 10% of water by weight with respect to the total weight of the composition.

**15.** Method for making up keratinous fibres, such as the eyelashes or eyebrows, which consists in applying the make-up composition according to any one of the preceding claims from the base towards the free end of the eyelashes so as to elongate the said keratinous fibres and in particular the outer part of the fringe of eyelashes.

**16.** Packaging and applicational combination for a composition for making up keratinous fibres, comprising:

i) a reservoir,
ii) a composition for making up keratinous fibres according to any one of Claims 1 to 14, positioned inside the reservoir, the said composition exhibiting a dmax threading nature of greater than or equal to 5 mm, and
iii) a device for the application of the make-up and/or care composition.

Europäisches Patentamt
European Patent Office
Office européen des brevets

# PARTIAL EUROPEAN SEARCH REPORT

under Rule 62a and/or 63 of the European Patent Convention. This report shall be considered, for the purposes of subsequent proceedings, as the European search report

Application Number
EP 13 17 6937

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/252698 A1 (FENG SUE [US] ET AL) 8 October 2009 (2009-10-08) | 1-4,8-14 | INV. A61K8/31 A61K8/73 A61K8/81 A61K8/85 A61K8/891 A61K8/893 A61K8/90 A61Q1/10 |
| Y | * paragraphs [0002], [0009], [0054], [0055], [0070]; claims; example 1 * | 1-16 | |
| X,D | EP 1 213 316 A2 (SHINETSU CHEMICAL CO [JP]) 12 June 2002 (2002-06-12) | 1-5, 11-13 | |
| Y | * paragraphs [0023] - [0040]; claims; examples 12,13 * | 1-16 | |
| Y | FR 2 905 068 A1 (OREAL [FR]) 29 February 2008 (2008-02-29) * claims * | 1-16 | |

TECHNICAL FIELDS SEARCHED (IPC)

A61K
A61Q

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

1

| Place of search | Date of completion of the search | Examiner |
|---|---|---|
| Munich | 21 February 2014 | Pregetter, Magdalena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

Europäisches Patentamt
European Patent Office
Office européen des brevets

**INCOMPLETE SEARCH SHEET C**

Application Number
EP 13 17 6937

Claim(s) searched incompletely:
1-16

Reason for the limitation of the search:

The independent claims of the present application define (inter alia) an unusual parameter "dmax threading nature".

The use of this unusual parameter in the context of a claim defined by greatly varying ingredients (silicone polymers of general formula (I'), "compounds capable of conferring on the composition said dmax threading nature, optional compounds) and the lack of a precise method of measurement for said unusual parameter (according to the description the method has been modified and no temperature of the surrounding environment which seems to be crucial for the results is defined) renders the subject-matter of all claims unclear (Art 84 EPC).

Also the criteria for selecting the appropriate compounds leading to a certain "dmax threading nature" have not been disclosed.
The compound capable of conferring the certain "dmax threading nature" to the composition can be selected from various groups of molecules: certain types of polymers (e.g. any ethylene-based copolymer!), any silicone resin, non polymeric compounds (e.g. waxes), and compounds only defined by their physical appearance (fibres).
To this extremely broad group of molecules a silicone polymer falling within a general formula and further optional ingredients must be added.
There is no teaching in the application as filed as to which parameters, e.g. interaction of compounds due to charge, hydrophilicity, molecular weight, ..., have to be taken into consideration.
This complete lack of disclosure as to how an unusual parameter has to be achieved leads to a lack of disclosure (Art. 83 EPC).

The non-compliance with the substantive provisions (Art. 84 and 83 EPC) is such that a meaningful search of the whole claimed subject-matter of the claims can not be carried out. The search has been restricted to the most preferred silicone polymers and the most preferred "compound capable of conferring the dmax threading nature" as exemplified on pages 79 and 80 of the description.

**ANNEX TO THE EUROPEAN SEARCH REPORT ON EUROPEAN PATENT APPLICATION NO.** EP 13 17 6937

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2014

| Patent document cited in search report | Publication date | Patent family member(s) | Publication date |
|---|---|---|---|
| US 2009252698 A1 | 08-10-2009 | NONE | |
| EP 1213316 A2 | 12-06-2002 | DE 60117734 T2 | 16-11-2006 |
| | | EP 1213316 A2 | 12-06-2002 |
| | | JP 3976226 B2 | 12-09-2007 |
| | | JP 2002179798 A | 26-06-2002 |
| | | US 2002131947 A1 | 19-09-2002 |
| FR 2905068 A1 | 29-02-2008 | CN 101505718 A | 12-08-2009 |
| | | EP 2079437 A1 | 22-07-2009 |
| | | FR 2905068 A1 | 29-02-2008 |
| | | JP 2010501530 A | 21-01-2010 |
| | | KR 20090046859 A | 11-05-2009 |
| | | US 2010021406 A1 | 28-01-2010 |
| | | WO 2008023056 A1 | 28-02-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1430868 A
- FR 2905068
- EP 1411069 A
- WO 04028488 A
- WO 04028493 A
- FR 2792190 A
- EP 1213316 A
- EP 1416016 A
- EP 1424373 A
- WO 06013413 A
- WO 2007068371 A, Cognis
- WO 2008155059 A
- EP 847752 A
- FR 2232303 A
- US 5162410 A
- WO 2004073626 A
- WO 04055081 A
- US 6009884 A
- US 5853010 A
- US 6220252 B


### Non-patent literature cited in the description


- Handbook of Pressure Sensitive Adhesives. 1989, 609-619
- Polymer Handbook. John Wiley, 1989
- **GRIFFIN.** *J. Soc. Cosm. Chem.,* 1954, vol. 5, 249-256
- Encyclopedia of Chemical Technology, Kirk-Othmer. Wiley, 1979, vol. 22, 333-432